# EUROPEAN PATENT APPLICATION

(11) **EP 2 377 551 A2**
(43) Date of publication of application: **19.10.2011**
(21) Application number: 11162713.9
(22) Date of filing: 06.11.2006
(51) Int. Cl.: A61K 39/145, A61K 39/39

(54) **Adjuvanted influenza vaccines including cytokine-inducing agents**

(30) Priority: 04.11.2005 US 734026 P; 11.11.2005 US 735274 P
(62) Divisional of application: 06808426.8
(71) Applicant: Novartis Vaccines and Diagnostics S.r.l., 53100 Siena (SI) (IT)
(72) Inventor: Rappuoli, Rino, 53100 Siena (IT); O'Hagan, Derek, 53100 Siena (IT); Del Giudice, Giuseppe, 53100 Siena (IT)
(74) Representative: Marshall, Cameron John

(57) **Abstract**

While oil-in-water emulsions are excellent adjuvants for influenza vaccines, their efficacy can be improved by additionally including other immunostimulating agent(s) to improve cytokine responses, such as γ-interferon response. Thus a vaccine comprises: (i) an influenza virus antigen; (ii) an oil-in-water emulsion adjuvant; and (iii) a cytokine-inducing agent, wherein the cytokine inducing agent is an agonist of human TLR4.

## Description

### TECHNICAL FIELD

This invention is in the field of adjuvanted vaccines for protecting against influenza virus infection.

### BACKGROUND ART

Influenza vaccines currently in general use do not include an adjuvant. These vaccines are described in more detail in chapters 17 & 18 of reference 1. They are based on live virus or inactivated virus, and inactivated vaccines can be based on whole virus, 'split' virus or on purified surface antigens (including haemagglutinin and neuraminidase). Haemagglutinin (HA) is the main immunogen in inactivated influenza vaccines, and vaccine doses are standardized by reference to HA levels, with vaccines typically containing about 15µg of HA per strain.

In a pandemic influenza outbreak then a large number of doses of influenza vaccine will be needed, but it will be difficult to increase vaccine supply to meet the huge demand. Rather than produce more vaccine antigen, therefore, it has been proposed to use a lower amount of antigen per strain, and to use an adjuvant to compensate for the reduced antigen dose. It has also been proposed to use the same approach in inter-pandemic periods *e.g*. to allow greater coverage of the population without increasing manufacturing levels.

The use of aluminum salt adjuvants has been suggested for influenza vaccines (*e.g*. see refs 2-5). The use of the MF59 oil-in-water emulsion has also been reported [6], and this emulsion is also found in the commercial FLUAD™ product from Chiron Vaccines.

It is an object of the invention to provide further and improved adjuvanted influenza vaccines (for both pandemic and interpandemic use) and methods for their preparation.

### DISCLOSURE OF THE INVENTION

It has now been found that, while oil-in-water emulsions are excellent adjuvants for influenza vaccines, their efficacy can be improved by additionally including other immunostimulating agent(s). Rather than merely increasing haemagglutination titers or anti-haemagglutinin ELISA titers, which are measures of the *quantity* of an immune response, the effect of the additional agent(s) is to increase the *quality* of the response. In particular, the additional agents have been found to improve the cytokine responses elicited by influenza vaccines, such as the interferon-γ response, with the improvement being much greater than seen when either the adjuvant or the agent is used on its own. Cytokine responses are known to be involved in the early and decisive stages of host defense against influenza infection [7].

Therefore the invention provides an immunogenic composition comprising: (i) an influenza virus antigen; (ii) an oil-in-water emulsion adjuvant; and (iii) a cytokine-inducing agent.

The invention also provides a method for preparing an immunogenic composition comprising the steps of combining: (i) an influenza virus antigen; (ii) an oil-in-water emulsion adjuvant; and (iii) a cytokine-inducing agent.

The invention provides a kit comprising: (i) a first kit component comprising an influenza virus antigen; and (ii) a second kit component comprising an oil-in-water emulsion adjuvant, wherein either (a) the first component or the second component includes a cytokine-inducing agent, or (b) the kit includes a third kit component comprising a cytokine-inducing agent.

### The oil-in-water emulsion adjuvant

Oil-in-water emulsions have been found to be particularly suitable for use in adjuvanting influenza virus vaccines. Various such emulsions are known, and they typically include at least one oil and at least one surfactant, with the oil(s) and surfactant(s) being biodegradable (metabolisable) and biocompatible. The oil droplets in the emulsion are generally less than 5µm in diameter, and may even have a sub-micron diameter, with these small sizes being achieved with a microfluidiser to provide stable emulsions. Droplets with a size less than 220nm are preferred as they can be subjected to filter sterilization.

The invention can be used with oils such as those from an animal (such as fish) or vegetable source. Sources for vegetable oils include nuts, seeds and grains. Peanut oil, soybean oil, coconut oil, and olive oil, the most commonly available, exemplify the nut oils. Jojoba oil can be used e.g. obtained from the jojoba bean. Seed oils include safflower oil, cottonseed oil, sunflower seed oil, sesame seed oil and the like. In the grain group, corn oil is the most readily available, but the oil of other cereal grains such as wheat, oats, rye, rice, teff, triticale and the like may also be used. 6-10 carbon fatty acid esters of glycerol and 1,2-propanediol, while not occurring naturally in seed oils, may be prepared by hydrolysis, separation and esterification of the appropriate materials starting from the nut and seed oils. Fats and oils from mammalian milk are metabolizable and may therefore be used in the practice of this invention. The procedures for separation, purification, saponification and other means necessary for obtaining pure oils from animal sources are well known in the art. Most fish contain metabolizable oils which may be readily recovered. For example, cod liver oil, shark liver oils, and whale oil such as spermaceti exemplify several of the fish oils which may be used herein. A number of branched chain oils are synthesized biochemically in 5-carbon isoprene units and are generally referred to as terpenoids. Shark liver oil contains a branched, unsaturated terpenoids known as squalene, 2,6,10,15,19,23-hexamethyl-2,6,10,14,18,22-tetracosahexaene, which is particularly preferred herein. Squalane, the saturated analog to squalene, is also a preferred oil. Fish oils, including squalene and squalane, are readily available from commercial sources or may be obtained by methods known in the art. Other preferred oils are the tocopherols (see below). Mixtures of oils can be used.

Surfactants can be classified by their 'HLB' (hydrophile/lipophile balance). Preferred surfactants of the invention have a HLB of at least 10, preferably at least 15, and more preferably at least 16. The invention can be used with surfactants including, but not limited to: the polyoxyethylene sorbitan esters surfactants (commonly referred to as the Tweens), especially polysorbate 20 and polysorbate 80; copolymers of ethylene oxide (EO), propylene oxide (PO), and/or butylene oxide (BO), sold under the DOWFAX™ tradename, such as linear EO/PO block copolymers; octoxynols, which can vary in the number of repeating ethoxy (oxy-1,2-ethanediyl) groups, with octoxynol-9 (Triton X-100, or t-octylphenoxypolyethoxyethanol) being of particular interest; (octylphenoxy)polyethoxyethanol (IGEPAL CA-630/NP-40); phospholipids such as phosphatidylcholine (lecithin); nonylphenol ethoxylates, such as the Tergitol™ NP series; polyoxyethylene fatty ethers derived from lauryl, cetyl, stearyl and oleyl alcohols (known as Brij surfactants), such as triethyleneglycol monolauryl ether (Brij 30); and sorbitan esters (commonly known as the SPANs), such as sorbitan trioleate (Span 85) and sorbitan monolaurate. Non-ionic surfactants are preferred. Preferred surfactants for including in the emulsion are Tween 80 (polyoxyethylene sorbitan monooleate), Span 85 (sorbitan trioleate), lecithin and Triton X-100.

Mixtures of surfactants can be used *e.g*. Tween 80/Span 85 mixtures. A combination of a polyoxyethylene sorbitan ester such as polyoxyethylene sorbitan monooleate (Tween 80) and an octoxynol such as t-octylphenoxypolyethoxyethanol (Triton X-100) is also suitable. Another useful combination comprises laureth 9 plus a polyoxyethylene sorbitan ester and/or an octoxynol.

Preferred amounts of surfactants (% by weight) are: polyoxyethylene sorbitan esters (such as Tween 80) 0.01 to 1%, in particular about 0.1 %; octyl- or nonylphenoxy polyoxyethanols (such as Triton X-100, or other detergents in the Triton series) 0.001 to 0.1 %, in particular 0.005 to 0.02%; polyoxyethylene ethers (such as laureth 9) 0.1 to 20 %, preferably 0.1 to 10 % and in particular 0.1 to 1 % or about 0.5%.

Specific oil-in-water emulsion adjuvants useful with the invention include, but are not limited to:
- A submicron emulsion of squalene, Tween 80, and Span 85. The composition of the emulsion by volume can be about 5% squalene, about 0.5% polysorbate 80 and about 0.5% Span 85. In weight terms, these ratios become 4.3% squalene, 0.5% polysorbate 80 and 0.48% Span 85. This adjuvant is known as 'MF59' [8-10], as described in more detail in Chapter 10 of ref. 11 and chapter 12 of ref. 12. The MF59 emulsion advantageously includes citrate ions *e.g*. 10mM sodium citrate buffer.
- An emulsion of squalene, a tocopherol, and Tween 80. The emulsion may include phosphate buffered saline. It may also include Span 85 (*e.g*. at 1%) and/or lecithin. These emulsions may have from 2 to 10% squalene, from 2 to 10% tocopherol and from 0.3 to 3% Tween 80, and the weight ratio of squalene:tocopherol is preferably ≤1 as this provides a more stable emulsion. Squalene and Tween 80 may be present volume ratio of about 5:2. One such emulsion can be made by dissolving Tween 80 in PBS to give a 2% solution, then mixing 90ml of this solution with a mixture of (5g of DL-α-tocopherol and 5ml squalene), then microfluidising the mixture. The resulting emulsion may have submicron oil droplets *e.g*. with an average diameter of between 100 and 250nm, preferably about 180nm.

- An emulsion of squalene, a tocopherol, and a Triton detergent (*e.g*. Triton X-100). The emulsion may also include a 3d-MPL (see below). The emulsion may contain a phosphate buffer.
- An emulsion comprising a polysorbate (*e.g*. polysorbate 80), a Triton detergent (*e.g*. Triton X-100) and a tocopherol (*e.g*. an α-tocopherol succinate). The emulsion may include these three components at a mass ratio of about 75:11:10 (*e.g*. 750µg/ml polysorbate 80, 110µg/ml Triton X-100 and 100µg/ml α-tocopherol succinate), and these concentrations should include any contribution of these components from antigens. The emulsion may also include squalene. The emulsion may also include a 3d-MPL (see below). The aqueous phase may contain a phosphate buffer.
- An emulsion of squalane, polysorbate 80 and poloxamer 401 ("Pluronic™ L121"). The emulsion can be formulated in phosphate buffered saline, pH 7.4. This emulsion is a useful delivery vehicle for muramyl dipeptides, and has been used with threonyl-MDP in the "SAF-1" adjuvant [13] (0.05-1% Thr-MDP, 5% squalane, 2.5% Pluronic L121 and 0.2% polysorbate 80). It can also be used without the Thr-MDP, as in the "AF" adjuvant [14] (5% squalane, 1.25% Pluronic L121 and 0.2% polysorbate 80). Microfluidisation is preferred.
- An emulsion having from 0.5-50% of an oil, 0.1-10% of a phospholipid, and 0.05-5% of a non-ionic surfactant. As described in reference 15, preferred phospholipid components are phosphatidylcholine, phosphatidylethanolamine, phosphatidylserine, phosphatidylinositol, phosphatidylglycerol, phosphatidic acid, sphingomyelin and cardiolipin. Submicron droplet sizes are advantageous.
- A submicron oil-in-water emulsion of a non-metabolisable oil (such as light mineral oil) and at least one surfactant (such as lecithin, Tween 80 or Span 80). Additives may be included, such as QuilA saponin, cholesterol, a saponin-lipophile conjugate (such as GPI-0100, described in reference 16, produced by addition of aliphatic amine to desacylsaponin via the carboxyl group of glucuronic acid), dimethyidioctadecylammonium bromide and/or N,N-dioctadecyl-N,N-bis (2-hydroxyethyl)propanediamine.
- An emulsion in which a saponin (*e.g*. QuilA or QS21) and a sterol *(e.g.* a cholesterol) are associated as helical micelles [17].
- An emulsion comprising a mineral oil, a non-ionic lipophilic ethoxylated fatty alcohol, and a non-ionic hydrophilic surfactant (*e.g*. an ethoxylated fatty alcohol and/or polyoxyethylene-polyoxypropylene block copolymer) [18].
- An emulsion comprising a mineral oil, a non-ionic hydrophilic ethoxylated fatty alcohol, and a non-ionic lipophilic surfactant (*e.g*. an ethoxylated fatty alcohol and/or polyoxyethylene-polyoxypropylene block copolymer) [18].

The emulsions are preferably mixed with antigen extemporaneously, at the time of delivery. Thus the adjuvant and antigen are typically kept separately in a packaged or distributed vaccine, ready for final formulation at the time of use. The antigen will generally be in an aqueous form, such that the vaccine is finally prepared by mixing two liquids. The volume ratio of the two liquids for mixing can vary (*e.g*. between 5:1 and 1:5) but is generally about 1:1.

After the antigen and adjuvant have been mixed, haemagglutinin antigen will generally remain in aqueous solution but may distribute itself around the oil/water interface. In general, little if any haemagglutinin will enter the oil phase of the emulsion.

Where a composition includes a tocopherol, any of the α, β, γ, δ, ε or ξ tocopherols can be used, but α-tocopherols are preferred. The tocopherol can take several forms *e.g.* different salts and/or isomers. Salts include organic salts, such as succinate, acetate, nicotinate, *etc.* D-α-tocopherol and DL-α-tocopherol can both be used. Tocopherols are advantageously included in vaccines for use in elderly patients (*e.g*. aged 60 years or older) because vitamin E has been reported to have a positive effect on the immune response in this patient group [19] and a significant impact on the expression of genes involved in the Th1/Th2 balance [20]. They also have antioxidant properties that may help to stabilize the emulsions [21]. A preferred α-tocopherol is DL-α-tocopherol, and the preferred salt of this tocopherol is the succinate. The succinate salt has been found to cooperate with TNF-related ligands *in vivo.* Moreover, α-tocopherol succinate is known to be compatible with influenza vaccines and to be a useful preservative as an alternative to mercurial compounds [88].

### The cytokine-inducing agent

Compositions of the invention include a cytokine-inducing agent, and it has been found that the combination of this agent with an oil-in-water emulsion gives a surprisingly effective immunogenic composition, with a synergistic effect on T cell responses. T cell responses are reported to be better able than antibody responses to resist influenza virus antigenic drift. Moreover, T cell effector mechanisms may be an important determinant of vaccine-induced protection against serious illness in elderly patients [22], and it may be possible to diminish age-related susceptibility to influenza by inducing a more potent interferon-γ response [23].

The cytokine-inducing agents for inclusion in compositions of the invention are able, when administered to a patient, to elicit the immune system to release cytokines, including interferons and interleukins. Preferred agents can elicit the release of one or more of: interferon-γ; interleukin-1; interleukin-2; interleukin-12; TNF-α; TNF-β; and GM-CSF. Preferred agents elicit the release of cytokines associated with a Th1-type immune response e.g. interferon-γ, TNF-α, interleukin-2. Stimulation of both interferon-γ and interleukin-2 is preferred.

As a result of receiving a composition of the invention, therefore, a patient will have T cells that, when stimulated with an influenza antigen, will release the desired cytokine(s) in an antigen-specific manner. For example, T cells purified form their blood will release γ-interferon when exposed *in vitro* to influenza virus haemagglutinin. Methods for measuring such responses in peripheral blood mononuclear cells (PBMC) are known in the art, and include ELISA, ELISPOT, flow-cytometry and real-time PCR. For example, reference 24 reports a study in which antigen-specific T cell-mediated immune responses against tetanus toxoid, specifically γ-interferon responses, were monitored, and found that ELISPOT was the most sensitive method to discriminate antigen-specific TT-induced responses from spontaneous responses, but that intracytoplasmic cytokine detection by flow cytometry was the most efficient method to detect re-stimulating effects.

Suitable cytokine-inducing agents include, but are not limited to:
- An immunostimulatory oligonucleotide, such as one containing a CpG motif (a dinucleotide sequence containing an unmethylated cytosine linked by a phosphate bond to a guanosine), or a double-stranded RNA, or an oligonucleotide containing a palindromic sequence, or an oligonucleotide containing a poly(dG) sequence.
- 3-O-deacylated monophosphoryl lipid A ('3dMPL', also known as 'MPL™') [25-28].
- An imidazoquinoline compound, such as Imiquimod ("R837") [29,30], Resiquimod ("R-848") [31], and their analogs; and salts thereof (*e.g*. the hydrochloride salts). Further details about immunostimulatory imidazoquinolines can be found in references 32 to 36.
- A thiosemicarbazone compound, such as those disclosed in reference 37. Methods of formulating, manufacturing, and screening for active compounds are also described in reference 37. The thiosemicarbazones are particularly effective in the stimulation of human peripheral blood mononuclear cells for the production of cytokines, such as TNF-α.
- A tryptanthrin compound, such as those disclosed in reference 38. Methods of formulating, manufacturing, and screening for active compounds are also described in reference 38. The thiosemicarbazones are particularly effective in the stimulation of human peripheral blood mononuclear cells for the production of cytokines, such as TNF-α.
- A nucleoside analog, such as: (a) Isatorabine (ANA-245; 7-thia-8-oxoguanosine): 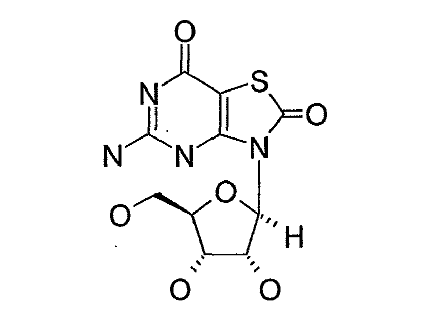 and prodrugs thereof; (b)ANA975; (c) ANA-025-1; (d) ANA380; (e) the compounds disclosed in references 39 to 41; (f) a compound having the formula: 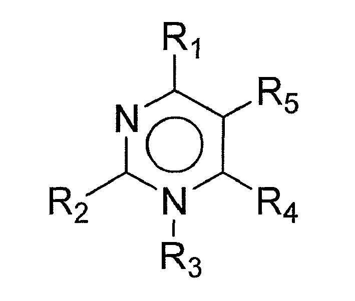 wherein:
   R₁ and R₂ are each independently H, halo, -NRₐR_{b}, -OH, C₁₋₆ alkoxy, substituted C₁₋₆ alkoxy, heterocyclyl, substituted heterocyclyl, C₆₋₁₀ aryl, substituted C₆₋₁₀ aryl, C₁₋₆ alkyl, or substituted C₁₋₆ alkyl;
   R₃ is absent, H, C₁₋₆ alkyl, substituted C₁₋₆ alkyl, C₆₋₁₀ aryl, substituted C₆₋₁₀ aryl, heterocyclyl, or substituted heterocyclyl;
   R₄ and R₅ are each independently H, halo, heterocyclyl, substituted heterocyclyl, - C(O)-R_{d}, C₁₋₆ alkyl, substituted C₁₋₆ alkyl, or bound together to form a 5 membered ring as in R₄₋₅: 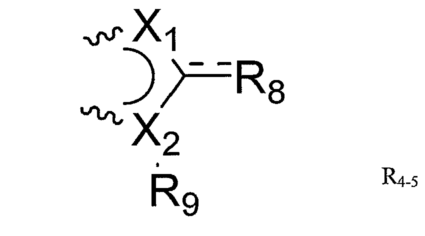 the binding being achieved at the bonds indicated by a 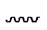
   X₁ and X₂ are each independently N, C, O, or S;
   R₈ is H, halo, -OH, C₁₋₆ alkyl, C₂₋₆ alkenyl, C₂₋₆ alkynyl, -OH, -NRₐR_{b}, -(CH₂)ₙ-O-R_{c}, -O-(C₁₋₆ alkyl), -S(O)ₚRₑ, or -C(O)-R_{d};
   R₉ is H, C₁₋₆ alkyl, substituted C₁₋₆ alkyl, heterocyclyl, substituted heterocyclyl or R₉ₐ, wherein R₉ₐ is: 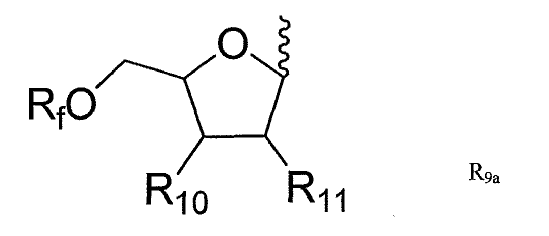 the binding being achieved at the bond indicated by a 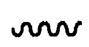
   R₁₀ and R₁₁ are each independently H, halo, C₁₋₆ alkoxy, substituted C₁₋₆ alkoxy, - NRₐR_{b}, or -OH;
      each Rₐ and R_{b} is independently H, C₁₋₆ alkyl, substituted C₁₋₆ alkyl, -C(O)R_{d}, C₆₋₁₀ aryl;
      each R_{c} is independently H, phosphate, diphosphate, triphosphate, C₁₋₆ alkyl, or substituted C₁₋₆ alkyl;
      each R_{d} is independently H, halo, C₁₋₆ alkyl, substituted C₁₋₆ alkyl, C₁₋₆ alkoxy, substituted C₁₋₆ alkoxy, -NH₂, -NH(C₁₋₆ alkyl), -NH(substituted C₁₋₆ alkyl), -N(C₁₋₆ alkyl)₂, -N(substituted C₁₋₆ alkyl)₂, C₆₋₁₀ aryl, or heterocyclyl;
      each Rₑ is independently H, C₁₋₆ alkyl, substituted C₁₋₆ alkyl, C₆₋₁₀ aryl, substituted C₆₋₁₀ aryl, heterocyclyl, or substituted heterocyclyl;
      each R_{f} is independently H, C₁₋₆ alkyl, substituted C₁₋₆ alkyl, -C(O)R_{d}, phosphate, diphosphate, or triphosphate;
      each n is independently 0, 1, 2, or 3;
      each p is independently 0, 1, or 2; or
   or (g) a pharmaceutically acceptable salt of any of (a) to (f), a tautomer of any of (a) to (f), or a pharmaceutically acceptable salt of the tautomer.
   - Loxoribine (7-allyl-8-oxoguanosine) [42].
   - Compounds disclosed in reference 43, including: Acylpiperazine compounds, Indoledione compounds, Tetrahydraisoquinoline (THIQ) compounds, Benzocyclodione compounds, Aminoazavinyl compounds, Aminobenzimidazole quinolinone (ABIQ) compounds [44,45], Hydrapthalamide compounds, Benzophenone compounds, Isoxazole compounds, Sterol compounds, Quinazilinone compounds, Pyrrole compounds [46], Anthraquinone compounds, Quinoxaline compounds, Triazine compounds, Pyrazalopyrimidine compounds, and Benzazole compounds [47].
   - A polyoxidonium polymer [48,49] or other N-oxidized polyethylene-piperazine derivative.
   - Compounds disclosed in reference 50.
   - A compound of formula I, II or III, or a salt thereof: 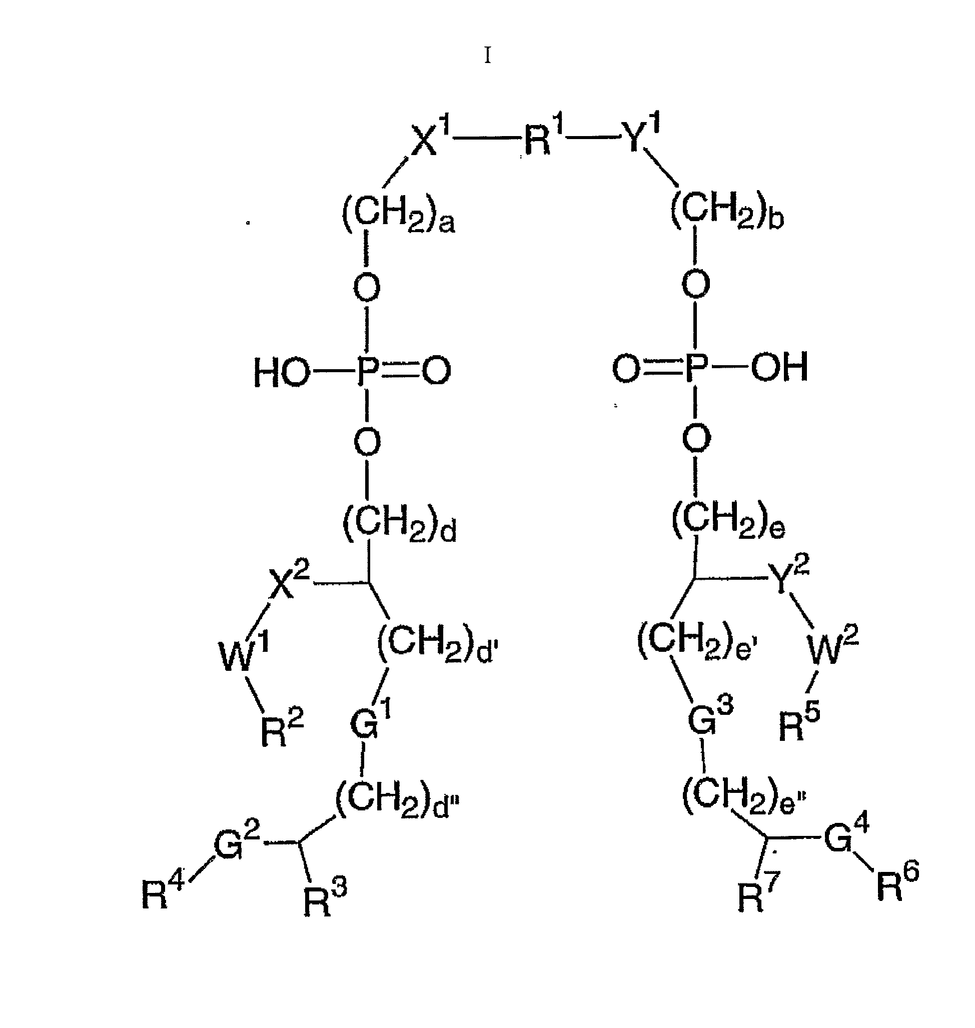 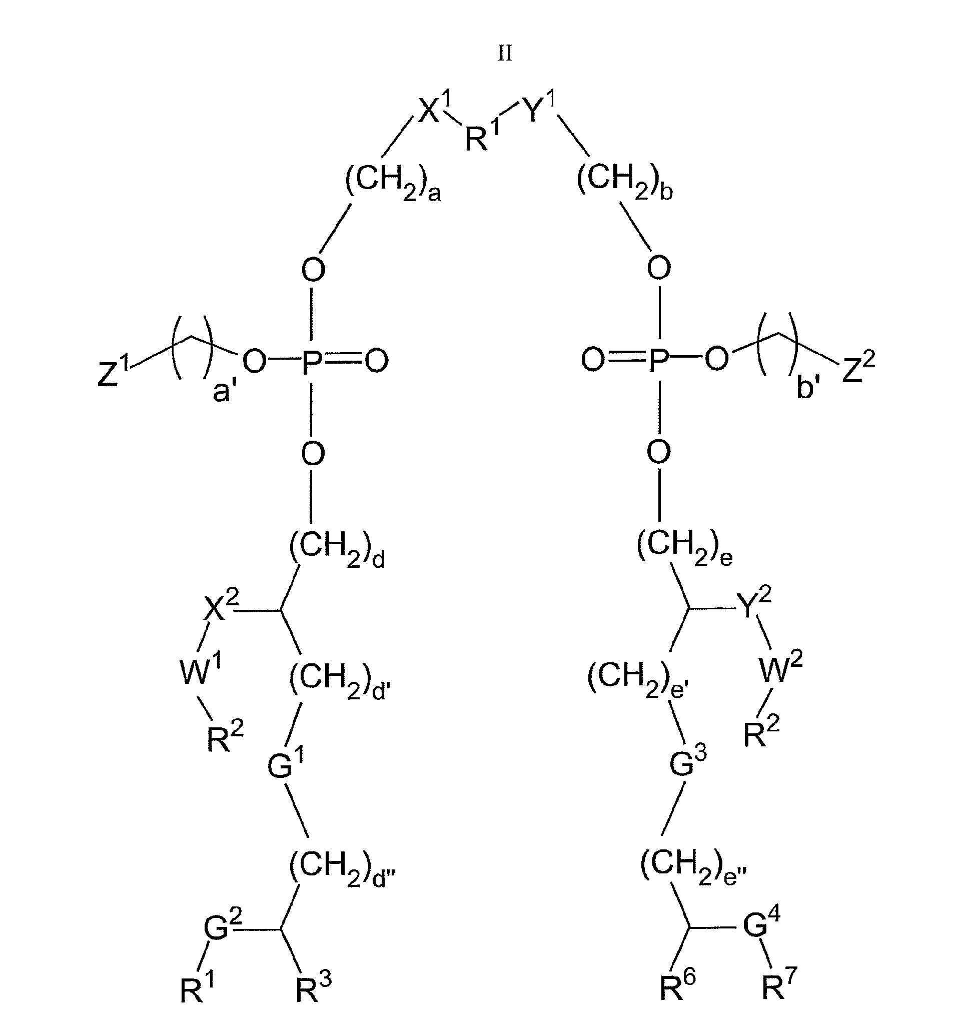 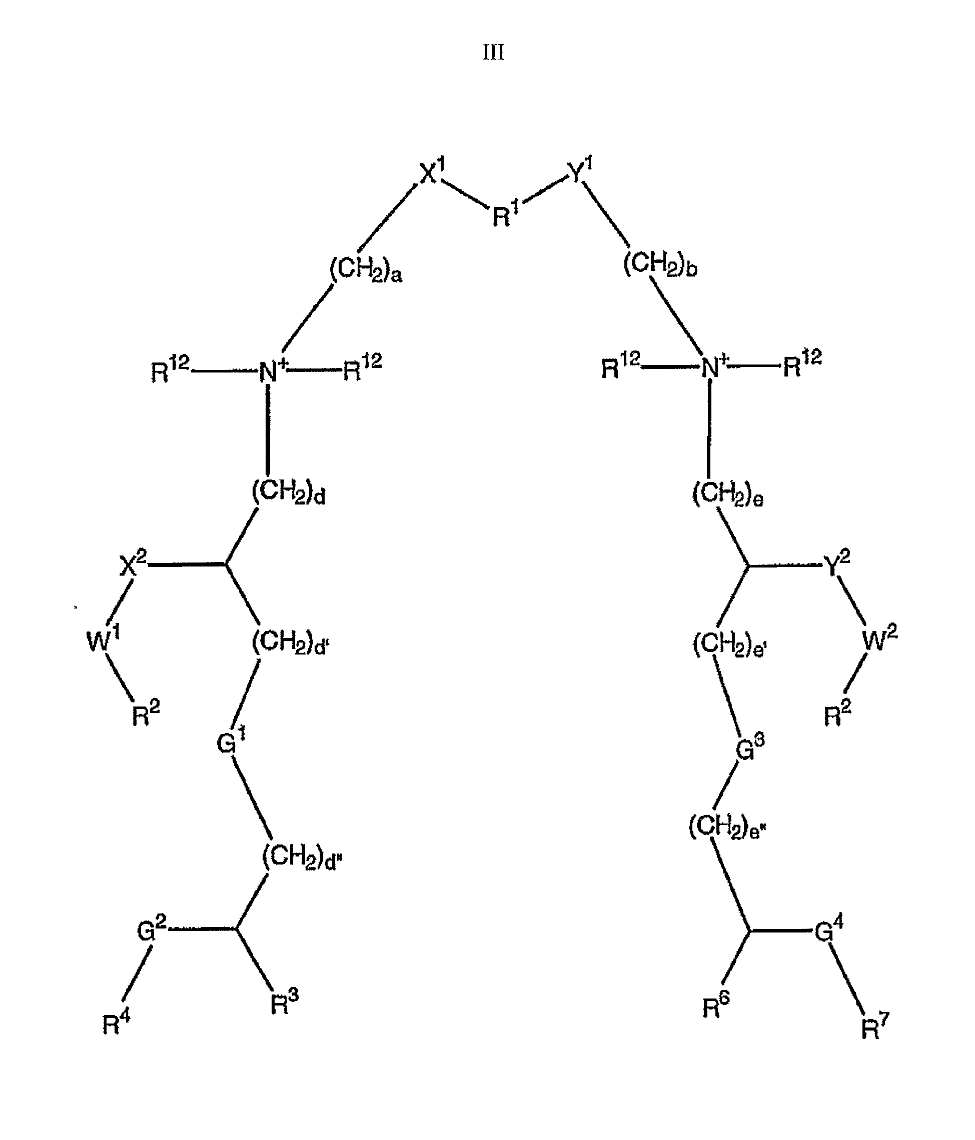 as defined in reference 51, such as 'ER 803058', 'ER 803732', 'ER 804053', ER 804058', 'ER 804059', 'ER 804442', 'ER 804680', 'ER 804764', 'ER 804057' (structure shown below): 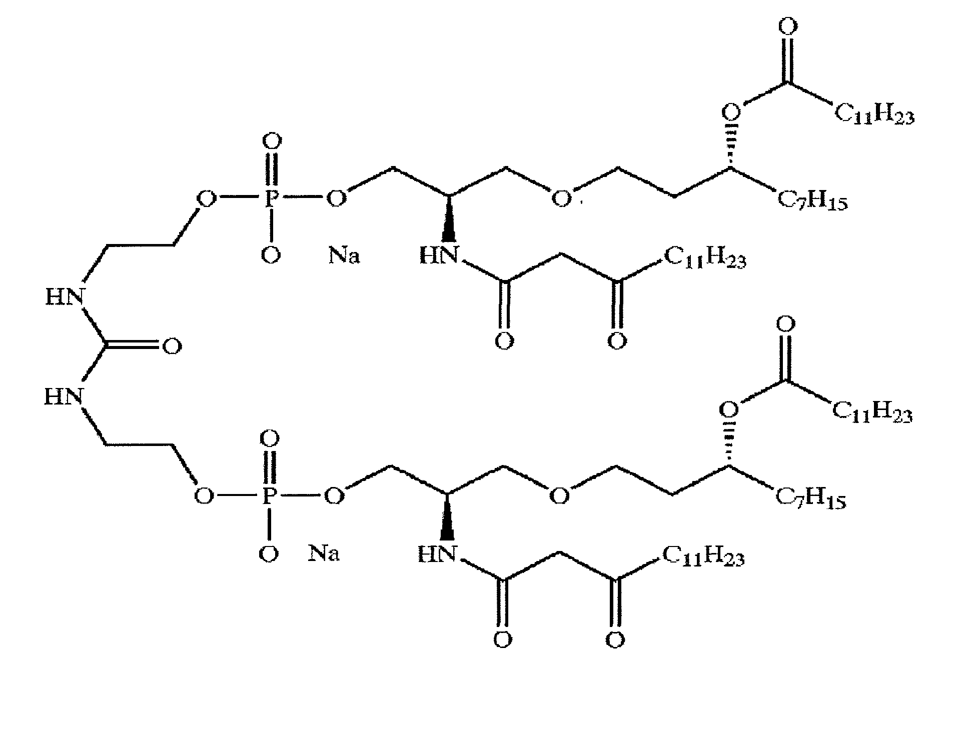 or ER-803022 (structure shown below): 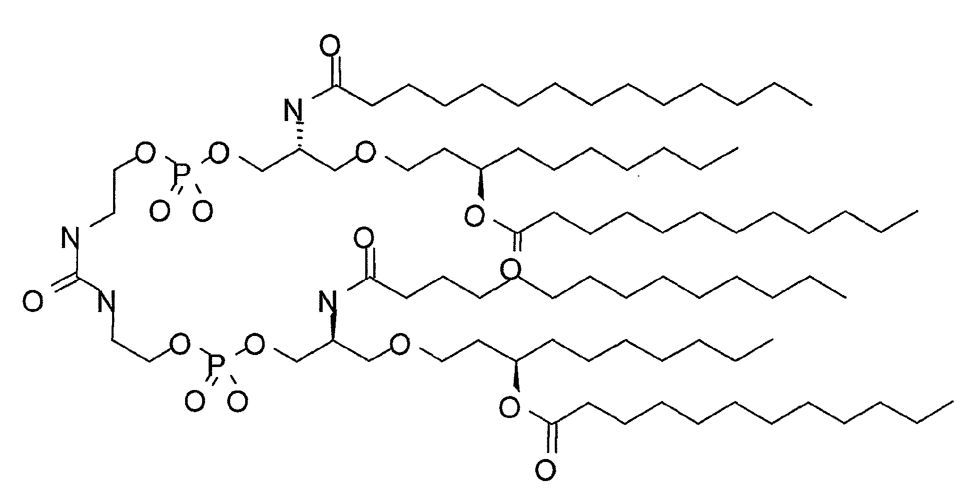
   - An aminoalkyl glucosaminide phosphate derivative, such as RC-529 [52,53]. The ability of RC-529 to stimulate cytokine responses in CD4⁺ T cells is reported in reference 54.
   - A phosphazene, such as poly[di(carboxylatophenoxy)phosphazene] ("PCPP") as described, for example, in references 55 and 56.
   - Compounds containing lipids linked to a phosphate-containing acyclic backbone, such as the TLR4 antagonist E5564 [57,58]: 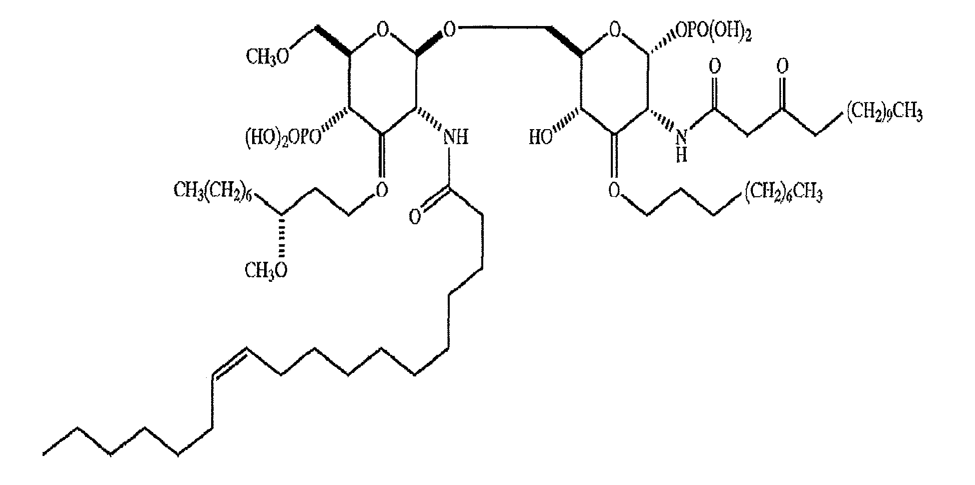
   - Small molecule immunopotentiators (SMIPs) such as:
      N2-methyl-1-(2-methylpropyl)-1H-imidazo[4,5-c]quinoline-2,4-diamine;
      N2,N2-dimethyl-1-(2-methylpropyl)-1H-imidazo[4,5-c]quinoline-2,4-diamine;
      N2-ethyl-N2-methyl-1-(2-methylpropyl)-1H-imidazo[4,5-c]quinoline-2,4-diamine;
      N2-methyl-1-(2-methylpropyl)-N2-propyl-1H-imidazo[4,5-c]quinoline-2,4-diamine;
      1-(2-methylpropyl)-N2-propyl-1H-imidazo[4,5-c]quinoline-2,4-diamine;
      N2-butyl-1-(2-methylpropyl)-1H-imidazo[4,5-c]quinoline-2,4-diamine;
      N2-butyl-N2-methyl-1-(2-methylpropyl)-1H-imidazo[4,5-c]quinoline-2,4-diamine;
      N2-methyl-1-(2-methylpropyl)-N2-pentyl-1H-imidazo[4,5-c]quinoline-2,4-diamine;
      N2-methyl-1-(2-methylpropyl)-N2-prop-2-enyl-1H-imidazo[4,5-c]quinoline-2,4-diamine;
      1-(2-methylpropyl)-2-[(phenylmethyl)thio]-1H-imidazo[4,5-c]quinolin-4-amine;
      1-(2-methylpropyl)-2-(propylthio)-1H-imidazo[4,5-c]quinolin-4-amine ;
      2-[[4-amino-1-(2-methylpropyl)-1H-imidazo[4,5-c]quinolin-2-yl](methyl)amino]ethanol;
      2-[[4-amino-1-(2-methylpropyl)-1H-imidazo[4,5-c]quinolin-2-yl](methyl)amino]ethyl acetate;
      4-amino-1-(2-methylpropyl)-1,3-dihydro-2H-imidazo[4,5-c]quinolin-2-one;
      N2-butyl-1-(2-methylpropyl)-N4,N4-bis(phenylmethyl)-1H-imidazo[4,5-c]quinoline-2,4-diamine;
      N2-butyl-N2-methyl-1-(2-methylpropyl)-N4,N4-bis(phenylmethyl)-1H-imidazo[4,5-c]quinoline-2,4-diamine;
      N2-methyl-1-(2-methylpropyl)-N4,N4-bis(phenylmethyl)-1H-imidazo[4,5-c]quinoline-2,4-diamine;
      N2,N2-dimethyl-1-(2-methylpropyl)-N4,N4-bis(phenylmethyl)-1H-imidazo[4,5-c]quinoline-2,4-diamine;
      1-{4-amino-2-[methyl(propyl)amino]-1H-imidazo[4,5-c]quinolin-1-yl }-2-methylpropan-2-ol;
      1-[4-amino-2-(propylamino)-1H-imidazo[4,5-c]quinolin-1-yl]-2-methylpropan-2-ol;
      N4,N4-dibenzyl-1-(2-methoxy-2-methylpropyl)-N2-propyl-1H-imidazo[4,5-c]quinoline-2,4-diamine.

The cytokine-inducing agents for use in the present invention may be modulators and/or agonists of Toll-Like Receptors (TLR). For example, they may be agonists of one or more of the human TLR1, TLR2, TLR3, TLR4, TLR7, TLR8, and/or TLR9 proteins. Preferred agents are agonists of TLR7 (*e.g*. imidazoquinilones) and/or TLR9 *(e.g.* CpG oligonucleotides). These agents are useful for activating innate immunity pathways.

The cytokine-inducing agent can be added to the composition at various stages during its production. For example, it may be within an antigen composition, and this mixture can then be added to an oil-in-water emulsion. As an alternative, it may be within an oil-in-water emulsion, in which case the agent can either be added to the emulsion components before emulsification, or it can be added to the emulsion after emulsification. Similarly, the agent may be coacervated within the emulsion droplets. The location and distribution of the cytokine-inducing agent within the final composition will depend on its hydrophilic/lipophilic properties *e.g*. the agent can be located in the aqueous phase, in the oil phase, and/or at the oil-water interface.

The cytokine-inducing agent can be conjugated to a separate agent, such as an antigen (*e.g*. CRM197). A general review of conjugation techniques for small molecules is provided in ref. 59. As an alternative, the adjuvants may be non-covalently associated with additional agents, such as by way of hydrophobic or ionic interactions.

Two preferred cytokine-inducing agents are (a) immunostimulatory oligonucleotides and (b) 3dMPL.

### Immunostimulatory oligonucleotides

Immunostimulatory oligonucleotides can include nucleotide modifications/analogs such as phosphorothioate modifications and can be double-stranded or (except for dsRNA) single-stranded. References 60, 61 and 62 disclose possible analog substitutions *e.g.* replacement of guanosine with 2'-deoxy-7-deazaguanosine. The adjuvant effect of CpG oligonucleotides is further discussed in refs. 63-68. The CpG sequence may be directed to TLR9, such as the motif GTCGTT or TTCGTT [69]. The CpG sequence may be specific for inducing a Th1 immune response, such as a CpG-A ODN (oligodeoxynucleotide), or it may be more specific for inducing a B cell response, such a CpG-B ODN. CpG-A and CpG-B ODNs are discussed in refs. 70-72. Preferably, the CpG is a CpG-A ODN. Preferably, the CpG oligonucleotide is constructed so that the 5' end is accessible for receptor recognition. Optionally, two CpG oligonucleotide sequences may be attached at their 3' ends to form "immunomers". See, for example, references 69 & 73-75. A useful CpG adjuvant is CpG7909, also known as ProMune™ (Coley Pharmaceutical Group, Inc.).

As an alternative, or in addition, to using CpG sequences, TpG sequences can be used [76]. These oligonucleotides may be free from unmethylated CpG motifs.

The immunostimulatory oligonucleotide may be pyrimidine-rich. For example, it may comprise more than one consecutive thymidine nucleotide (*e.g*. TTTT, as disclosed in ref. 76), and/or it may have a nucleotide composition with >25% thymidine (*e.g*. >35%, >40%, >50%, >60%, >80%, *etc.*). For example, it may comprise more than one consecutive cytosine nucleotide (*e.g*. CCCC, as disclosed in ref. 76), and/or it may have a nucleotide composition with >25% cytosine (*e.g*. >35%, >40%, >50%, >60%, >80%, *etc.*). These oligonucleotides may be free from unmethylated CpG motifs.

Immunostimulatory oligonucleotides will typically comprise at least 20 nucleotides. They may comprise fewer than 100 nucleotides.

### 3dMPL

3dMPL (also known as 3 de-O-acylated monophosphoryl lipid A or 3-O-desacyl-4'-monophosphoryl lipid A) is an adjuvant in which position 3 of the reducing end glucosamine in monophosphoryl lipid A has been de-acylated. 3dMPL has been prepared from a heptoseless mutant of *Salmonella minnesota,* and is chemically similar to lipid A but lacks an acid-labile phosphoryl group and a base-labile acyl group. It activates cells of the monocyte/macrophage lineage and stimulates release of several cytokines, including IL-1, IL-12, TNF-α and GM-CSF (see also ref. 54). Preparation of 3dMPL was originally described in reference 77.

3dMPL can take the form of a mixture of related molecules, varying by their acylation (*e.g*. having 3, 4, 5 or 6 acyl chains, which may be of different lengths). The two glucosamine (also known as 2-deoxy-2-amino-glucose) monosaccharides are N-acylated at their 2-position carbons (*i.e*. at positions 2 and 2'), and there is also O-acylation at the 3' position. The group attached to carbon 2 has formula -NH-CO-CH₂-CR¹R^{1'}. The group attached to carbon 2' has formula -NH-CO-CH₂-CR²R^{2'}. The group attached to carbon 3' has formula -O-CO-CH₂-CR³R^{3'}. A representative structure is:

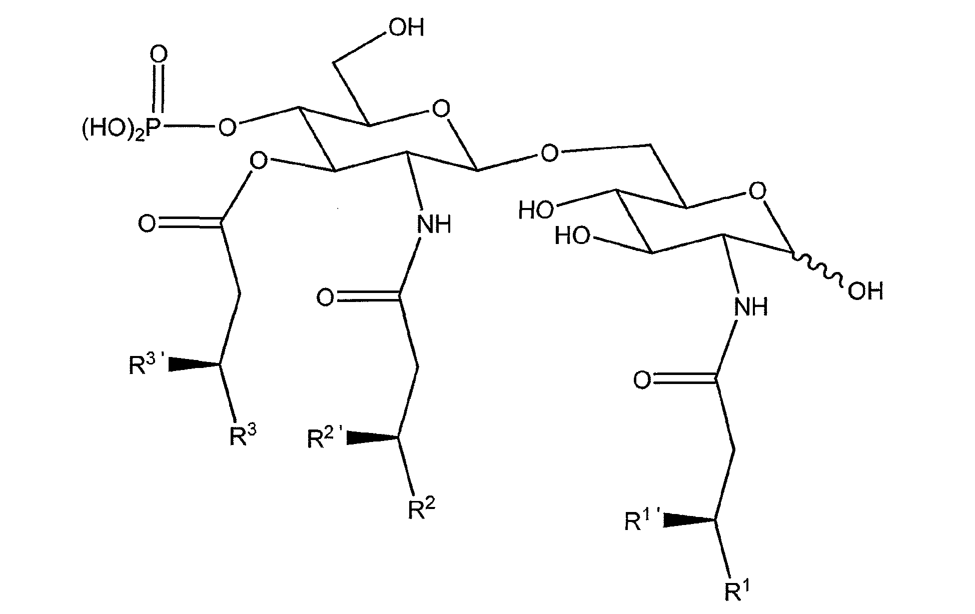

Groups R¹, R² and R³ are each independently -(CH₂)ₙ-CH₃. The value of *n* is preferably between 8 and 16, more preferably between 9 and 12, and is most preferably 10.

Groups R^{1'}, R^{2'} and R^{3'} can each independently be: (a) -H; (b) -OH; or (c) -O-CO-R⁴,where R⁴ is either -H or -(CH₂)ₘ-CH₃, wherein the value of *m* is preferably between 8 and 16, and is more preferably 10, 12 or 14. At the 2 position, *m* is preferably 14. At the 2' position, *m* is preferably 10. At the 3' position, *m* is preferably 12. Groups R^{1'}, R^{2'} and R^{3'} are thus preferably -O-acyl groups from dodecanoic acid, tetradecanoic acid or hexadecanoic acid.

When all of R^{1'}, R^{2'} and R^{3'} are -H then the 3dMPL has only 3 acyl chains (one on each of positions 2, 2' and 3'). When only two of R^{1'}, R^{2'} and R^{3'} are -H then the 3dMPL can have 4 acyl chains. When only one of R^{1'}, R^{2'} and R^{3'} is -H then the 3dMPL can have 5 acyl chains. When none of R^{1'}, R^{2'} and R^{3'} is -H then the 3dMPL can have 6 acyl chains. The 3dMPL adjuvant used according to the invention can be a mixture of these forms, with from 3 to 6 acyl chains, but it is preferred to include 3dMPL with 6 acyl chains in the mixture, and in particular to ensure that the hexaacyl chain form makes up at least 10% by weight of the total 3dMPL *e.g*. ≥20%, ≥30%, ≥40%, ≥50% or more. 3dMPL with 6 acyl chains has been found to be the most adjuvant-active form.

Thus the most preferred form of 3dMPL for inclusion in compositions of the invention is:

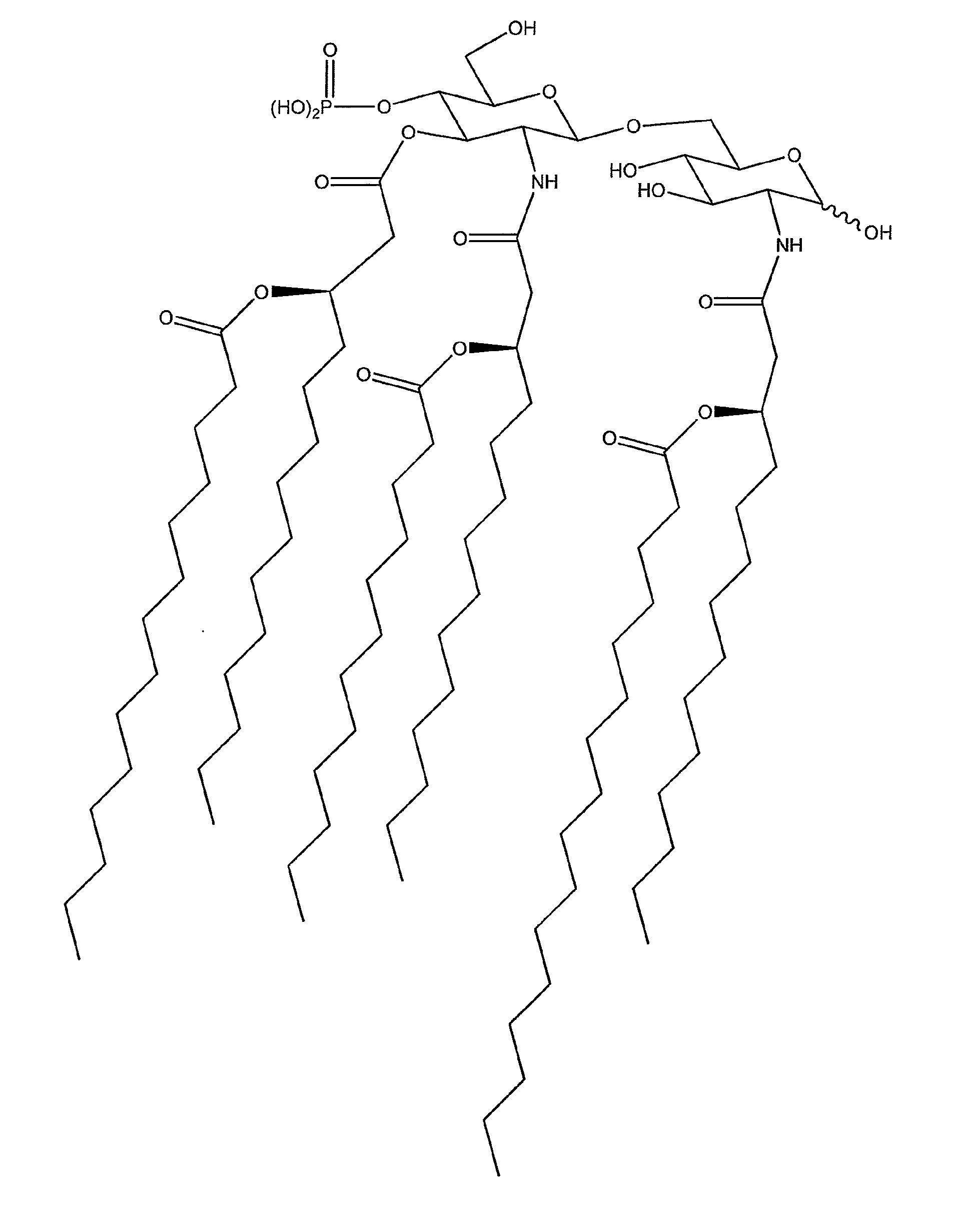

Where 3dMPL is used in the form of a mixture then references to amounts or concentrations of 3dMPL in compositions of the invention refer to the combined 3dMPL species in the mixture.

In aqueous conditions, 3dMPL can form micellar aggregates or particles with different sizes *e.g*. with a diameter <150nm or >500nm. Either or both of these can be used with the invention, and the better particles can be selected by routine assay. Smaller particles (*e.g*. small enough to give a clear aqueous suspension of 3dMPL) are preferred for use according to the invention because of their superior activity [78]. Preferred particles have a mean diameter less than 220nm, more preferably less than 200nm or less than 150nm or less than 120nm, and can even have a mean diameter less than 100nm. In most cases, however, the mean diameter will not be lower than 50nm. These particles are small enough to be suitable for filter sterilization. Particle diameter can be assessed by the routine technique of dynamic light scattering, which reveals a mean particle diameter. Where a particle is said to have a diameter of x nm, there will generally be a distribution of particles about this mean, but at least 50% by number (*e.g.* ≥60%, ≥70%, ≥80%, ≥90%, or more) of the particles will have a diameter within the range *x*±25%.

Substantially all of the 3dMPL is preferably located in the aqueous phase of the emulsion.

A typical amount of 3dMPL in a vaccine is 10-100µg/dose *e.g*. about 25µg or about 50µg.

The 3dMPL can be used on its own, or in combination with one or more further compounds. For example, it is known to use 3dMPL in combination with the QS21 saponin [79] (including in an emulsion [80]), with aluminum phosphate [81], or with aluminum hydroxide [82].

### The influenza virus antigen

Compositions of the invention include an influenza virus antigen. The antigen will typically be prepared from influenza virions but, as an alternative, antigens such as haemagglutinin can be expressed in a recombinant host (*e.g*. in an insect cell line using a baculovirus vector) and used in purified form [83,84]. In general, however, antigens will be from virions.

The antigen may take the form of a live virus or, more preferably, an inactivated virus. Chemical means for inactivating a virus include treatment with an effective amount of one or more of the following agents: detergents, formaldehyde, formalin, β-propiolactone, or UV light. Additional chemical means for inactivation include treatment with methylene blue, psoralen, carboxyfullerene (C60) or a combination of any thereof. Other methods of viral inactivation are known in the art, such as for example binary ethylamine, acetyl ethyleneimine, or gamma irradiation. The INFLEXAL™ product is a whole virion inactivated vaccine.

Where an inactivated virus is used, the vaccine may comprise whole virion, split virion, or purified surface antigens (including hemagglutinin and, usually, also including neuraminidase).

Virions can be harvested from virus-containing fluids by various methods. For example, a purification process may involve zonal centrifugation using a linear sucrose gradient solution that includes detergent to disrupt the virions. Antigens may then be purified, after optional dilution, by diafiltration.

Split virions are obtained by treating virions with detergents (*e.g.* ethyl ether, polysorbate 80, deoxycholate, tri-*N*-butyl phosphate, Triton X-100, Triton N101, cetyltrimethylammonium bromide, *etc*.) to produce subvirion preparations, including the 'Tween-ether' splitting process. Methods of splitting influenza viruses are well known in the art *e.g.* see refs. 85-90, *etc.* Splitting of the virus is typically carried out by disrupting or fragmenting whole virus, whether infectious or non-infectious with a disrupting concentration of a splitting agent. The disruption results in a full or partial solubilisation of the virus proteins, altering the integrity of the virus. Preferred splitting agents are non-ionic and ionic (*e.g*. cationic) surfactants *e.g*. alkylglycosides, alkylthioglycosides, acyl sugars, sulphobetaines, betains, polyoxyethylenealkylethers, N,N-dialkyl-Glucamides, Hecameg, alkylphenoxy-polyethoxyethanols, quaternary ammonium compounds, sarcosyl, CTABs (cetyl trimethyl ammonium bromides), tri-*N*-butyl phosphate, Cetavlon, myristyltrimethylammonium salts, lipofectin, lipofectamine, and DOT-MA, the octyl- or nonylphenoxy polyoxyethanols (*e.g*. the Triton surfactants, such as Triton X-100 or Triton N101), polyoxyethylene sorbitan esters (the Tween surfactants), polyoxyethylene ethers, polyoxyethlene esters, *etc.* One useful splitting procedure uses the consecutive effects of sodium deoxycholate and formaldehyde, and splitting can take place during initial virion purification (*e.g*. in a sucrose density gradient solution). Split virions can usefully be resuspended in sodium phosphate-buffered isotonic sodium chloride solution. The BEGRIVAC™, FLUARIX™, FLUZONE™ and FLUSHIELD™ products are split vaccines.

Purified surface antigen vaccines comprise the influenza surface antigens haemagglutinin and, typically, also neuraminidase. Processes for preparing these proteins in purified form are well known in the art. The FLUVIRIN™, AGRIPPAL™ and INFLUVAC™ products are subunit vaccines.

Influenza antigens can also be presented in the form of virosomes [91].

The influenza virus may be attenuated. The influenza virus may be temperature-sensitive. The influenza virus may be cold-adapted. These three possibilities apply in particular for live viruses.

Influenza virus strains for use in vaccines change from season to season. In the current inter-pandemic period, vaccines typically include two influenza A strains (H1N1 and H3N2) and one influenza B strain, and trivalent vaccines are typical. The invention may also use viruses from pandemic strains (*i*.*e*. strains to which the vaccine recipient and the general human population are immunologically naive), such as H2, H5, H7 or H9 subtype strains (in particular of influenza A virus), and influenza vaccines for pandemic strains may be monovalent or may be based on a normal trivalent vaccine supplemented by a pandemic strain. Depending on the season and on the nature of the antigen included in the vaccine, however, the invention may protect against one or more of influenza A virus HA subtypes H1, H2, H3, H4, H5, H6, H7, H8, H9, H10, H11, H12, H13, H14, H15 or H16. The invention may protect against one or more of influenza A virus NA subtypes N1, N2, N3, N4, N5, N6, N7, N8 or N9.

Other strains that can usefully be included in the compositions are strains which are resistant to antiviral therapy (*e.g*. resistant to oseltamivir [92] and/or zanamivir), including resistant pandemic strains [93].

The adjuvanted compositions of the invention are particularly useful for immunizing against pandemic strains. The characteristics of an influenza strain that give it the potential to cause a pandemic outbreak are: (a) it contains a new hemagglutinin compared to the hemagglutinins in currently-circulating human strains, *i.e*. one that has not been evident in the human population for over a decade *(e.g.* H2), or has not previously been seen at all in the human population (*e.g*. H5, H6 or H9, that have generally been found only in bird populations), such that the human population will be immunologically naïve to the strain's hemagglutinin; (b) it is capable of being transmitted horizontally in the human population; and (c) it is pathogenic to humans. A virus with H5 haemagglutinin type is preferred for immunising against pandemic influenza, such as a H5N1 strain. Other possible strains include H5N3, H9N2, H2N2, H7N1 and H7N7, and any other emerging potentially pandemic strains. Within the H5 subtype, a virus may fall into HA clade 1, HA clade 1', HA clade 2 or HA clade 3 [94], with clades 1 and 3 being particularly relevant.

Compositions of the invention may include antigen(s) from one or more (*e.g*. 1, 2, 3, 4 or more) influenza virus strains, including influenza A virus and/or influenza B virus. Where a vaccine includes more than one strain of influenza, the different strains are typically grown separately and are mixed after the viruses have been harvested and antigens have been prepared. Thus a process of the invention may include the step of mixing antigens from more than one influenza strain.

The influenza virus may be a reassortant strain, and may have been obtained by reverse genetics techniques. Reverse genetics techniques [*e.g*. 95-99] allow influenza viruses with desired genome segments to be prepared *in vitro* using plasmids. Typically, it involves expressing (a) DNA molecules that encode desired viral RNA molecules *e.g*. from polI promoters, and (b) DNA molecules that encode viral proteins *e.g*. from polII promoters, such that expression of both types of DNA in a cell leads to assembly of a complete intact infectious virion. The DNA preferably provides all of the viral RNA and proteins, but it is also possible to use a helper virus to provide some of the RNA and proteins. Plasmid-based methods using separate plasmids for producing each viral RNA are preferred [100-102], and these methods will also involve the use of plasmids to express all or some (*e.g*. just the FB1, PB2, PA and NP proteins) of the viral proteins, with 12 plasmids being used in some methods.

To reduce the number of plasmids needed, a recent approach [103] combines a plurality of RNA polymerase I transcription cassettes (for viral RNA synthesis) on the same plasmid (*e.g*. sequences encoding 1, 2, 3, 4, 5, 6, 7 or all 8 influenza A vRNA segments), and a plurality of protein-coding regions with RNA polymerase II promoters on another plasmid (*e.g*. sequences encoding 1, 2, 3,4, 5, 6, 7 or all 8 influenza A mRNA transcripts). Preferred aspects of the reference 103 method involve:
(a) PB 1, PB2 and PA mRNA-encoding regions on a single plasmid; and (b) all 8 vRNA-encoding segments on a single plasmid. Including the NA and HA segments on one plasmid and the six other segments on another plasmid can also facilitate matters.

As an alternative to using polI promoters to encode the viral RNA segments, it is possible to use bacteriophage polymerase promoters [104]. For instance, promoters for the SP6, T3 or T7 polymerases can conveniently be used. Because of the species-specificity of polI promoters, bacteriophage polymerase promoters can be more convenient for many cell types (*e.g*. MDCK), although a cell must also be transfected with a plasmid encoding the exogenous polymerase enzyme.

In other techniques it is possible to use dual poll and polII promoters to simultaneously code for the viral RNAs and for expressible mRNAs from a single template [105,106].

Thus an influenza A virus may include one or more RNA segments from a A/PR/8/34 virus (typically 6 segments from A/PR/8/34, with the HA and N segments being from a vaccine strain, *i.e.* a 6:2 reassortant), particularly when viruses are grown in eggs. It may also include one or more RNA segments from a A/WSN/33 virus, or from any other virus strain useful for generating reassortant viruses for vaccine preparation. Typically, the invention protects against a strain that is capable of human-to-human transmission, and so the strain's genome will usually include at least one RNA segment that originated in a mammalian (*e.g*. in a human) influenza virus. It may include NS segment that originated in an avian influenza virus.

The viruses used as the source of the antigens can be grown either on eggs (usually SPF eggs) or on cell culture. The current standard method for influenza virus growth uses embryonated hen eggs, with virus being purified from the egg contents (allantoic fluid). More recently, however, viruses have been grown in animal cell culture and, for reasons of speed and patient allergies, this growth method is preferred. If egg-based viral growth is used then one or more amino acids may be introduced into the allantoid fluid of the egg together with the virus [89].

The cell substrate will typically be a mammalian cell line. Suitable mammalian cells of origin include, but are not limited to, hamster, cattle, primate (including humans and monkeys) and dog cells. Various cell types may be used, such as kidney cells, fibroblasts, retinal cells, lung cells, *etc.* Examples of suitable hamster cells are the cell lines having the names BHK21 or HKCC. Suitable monkey cells are *e.g*. African green monkey cells, such as kidney cells as in the Vero cell line. Suitable dog cells are *e.g*. kidney cells, as in the MDCK cell line. Thus suitable cell lines include, but are not limited to: MDCK; CHO; 293T; BHK; Vero; MRC-5; PER.C6; WI-38; *etc..* Preferred mammalian cell lines for growing influenza viruses include: MDCK cells [107-110], derived from Madin Darby canine kidney; Vero cells [111-113], derived from African green monkey (*Cercopithecus aethiops*) kidney; or PER.C6 cells [114], derived from human embryonic retinoblasts. These cell lines are widely available *e.g*. from the American Type Cell Culture (ATCC) collection [115], from the Coriell Cell Repositories [116], or from the European Collection of Cell Cultures (ECACC). For example, the ATCC supplies various different Vero cells under catalog numbers CCL-81, CCL-81.2, CRL-1586 and CRL-1587, and it supplies MDCK cells under catalog number CCL-34. PER.C6 is available from the ECACC under deposit number 96022940. As a less-preferred alternative to mammalian cell lines, virus can be grown on avian cell lines [*e.g*. refs. 117-119], including cell lines derived from ducks (*e.g*. duck retina) or hens *e.g*. chicken embryo fibroblasts (CEF), *etc*. Examples include avian embryonic stem cells [117, 120], including the EBx cell line derived from chicken embryonic stem cells, EB45, EB 14, and EB 14-074 [121].

The most preferred cell lines for growing influenza viruses are MDCK cell lines. The original MDCK cell line is available from the ATCC as CCL-34, but derivatives of this cell line may also be used. For instance, reference 107 discloses a MDCK cell line that was adapted for growth in suspension culture ('MDCK 33016', deposited as DSM ACC 2219). Similarly, reference 122 discloses a MDCK-derived cell line that grows in suspension in serum-free culture ('B-702', deposited as FERM BP-7449). Reference 123 discloses non-tumorigenic MDCK cells, including 'MDCK-S' (ATCC PTA-6500), 'MDCK-SF101' (ATCC PTA-6501), 'MDCK-SF102' (ATCC PTA-6502) and 'MDCK-SF103' (PTA-6503). Reference 124 discloses MDCK cell lines with high susceptibility to infection, including 'MDCK.5F1' cells (ATCC CRL-12042). Any of these MDCK cell lines can be used.

Where virus has been grown on a mammalian cell line then the composition will advantageously be free from egg proteins (*e.g*. ovalbumin and ovomucoid) and from chicken DNA, thereby reducing allergenicity.

Where virus has been grown on a cell line then the culture for growth, and also the viral inoculum used to start the culture, will preferably be free from (*i.e.* will have been tested for and given a negative result for contamination by) herpes simplex virus, respiratory syncytial virus, parainfluenza virus 3, SARS coronavirus, adenovirus, rhinovirus, reoviruses, polyomaviruses, birnaviruses, circoviruses, and/or parvoviruses [125]. Absence of herpes simplex viruses is particularly preferred.

Where virus has been grown on a cell line then the composition preferably contains less than 10ng (preferably less than 1ng, and more preferably less than 100pg) of residual host cell DNA per dose, although trace amounts of host cell DNA may be present. In general, the host cell DNA that it is desirable to exclude from compositions of the invention is DNA that is longer than 100bp.

Measurement of residual host cell DNA is now a routine regulatory requirement for biologicals and is within the normal capabilities of the skilled person. The assay used to measure DNA will typically be a validated assay [126,127]. The performance characteristics of a validated assay can be described in mathematical and quantifiable terms, and its possible sources of error will have been identified. The assay will generally have been tested for characteristics such as accuracy, precision, specificity. Once an assay has been calibrated (*e.g*. against known standard quantities of host cell DNA) and tested then quantitative DNA measurements can be routinely performed. Three principle techniques for DNA quantification can be used: hybridization methods, such as Southern blots or slot blots [128]; immunoassay methods, such as the Threshold™ System [129]; and quantitative PCR [130]. These methods are all familiar to the skilled person, although the precise characteristics of each method may depend on the host cell in question *e.g*. the choice of probes for hybridization, the choice of primers and/or probes for amplification, *etc.* The Threshold™ system from *Molecular Devices* is a quantitative assay for picogram levels of total DNA, and has been used for monitoring levels of contaminating DNA in biopharmaceuticals [129]. A typical assay involves non-sequence-specific formation of a reaction complex between a biotinylated ssDNA binding protein, a urease-conjugated anti-ssDNA antibody, and DNA. All assay components are included in the complete Total DNA Assay Kit available from the manufacturer. Various commercial manufacturers offer quantitative PCR assays for detecting residual host cell DNA *e.g*. AppTec™ Laboratory Services, BioReliance™, Althea Technologies, *etc.* A comparison of a chemiluminescent hybridisation assay and the total DNA Threshold™ system for measuring host cell DNA contamination of a human viral vaccine can be found in reference 131.

Contaminating DNA can be removed during vaccine preparation using standard purification procedures *e.g.* chromatography, *etc.* Removal of residual host cell DNA can be enhanced by nuclease treatment *e.g*. by using a DNase. A convenient method for reducing host cell DNA contamination is disclosed in references 132 & 133, involving a two-step treatment, first using a DNase (*e.g*. Benzonase), which may be used during viral growth, and then a cationic detergent *(e.g.* CTAB), which may be used during virion disruption. Treatment with an alkylating agent, such as β-propiolactone, can also be used to remove host cell DNA, and advantageously may also be used to inactivate virions [134].

Vaccines containing <10ng *(e.g.* <1ng, <100pg) host cell DNA per 15µg of haemagglutinin are preferred, as are vaccines containing <10ng (*e.g*. <1ng, <100pg) host cell DNA per 0.25ml volume. Vaccines containing <10ng (*e.g*. <1ng, <100pg) host cell DNA per 50µg of haemagglutinin are more preferred, as are vaccines containing <10ng (*e.g*. <1ng, <100pg) host cell DNA per 0.5ml volume.

It is preferred that the average length of any residual host cell DNA is less than 500bp *e.g*. less than 400bp, less than 300bp, less than 200bp, less than 100bp, *etc.*

For growth on a cell line, such as on MDCK cells, virus may be grown on cells in suspension [107,135,136] or in adherent culture. One suitable MDCK cell line for suspension culture is MDCK 33016 (deposited as DSM ACC 2219). As an alternative, microcarrier culture can be used.

Cell lines supporting influenza virus replication are preferably grown in serum-free culture media and/or protein free media. A medium is referred to as a serum-free medium in the context of the present invention in which there are no additives from serum of human or animal origin. Protein-free is understood to mean cultures in which multiplication of the cells occurs with exclusion of proteins, growth factors, other protein additives and non-serum proteins, but can optionally include proteins such as trypsin or other proteases that may be necessary for viral growth. The cells growing in such cultures naturally contain proteins themselves.

Cell lines supporting influenza virus replication are preferably grown below 37°C [137] (*e.g*. 30-36°C, or at about 30°C, 31°C, 32°C, 33°C, 34°C, 35°C, 36°C) for example during viral replication.

The method for propagating virus in cultured cells generally includes the steps of inoculating the cultured cells with the strain to be cultured, cultivating the infected cells for a desired time period for virus propagation, such as for example as determined by virus titer or antigen expression (*e.g*. between 24 and 168 hours after inoculation) and collecting the propagated virus. The cultured cells are inoculated with a virus (measured by PFU or TCID₅₀) to cell ratio of 1:500 to 1:1, preferably 1:100 to 1:5, more preferably 1:50 to 1:10. The virus is added to a suspension of the cells or is applied to a monolayer of the cells, and the virus is absorbed on the cells for at least 60 minutes but usually less than 300 minutes, preferably between 90 and 240 minutes at 25°C to 40°C, preferably 28°C to 37°C. The infected cell culture (*e.g*. monolayers) may be removed either by freeze-thawing or by enzymatic action to increase the viral content of the harvested culture supernatants. The harvested fluids are then either inactivated or stored frozen. Cultured cells may be infected at a multiplicity of infection ("m.o.i.") of about 0.0001 to 10, preferably 0.002 to 5, more preferably to 0.001 to 2. Still more preferably, the cells are infected at a m.o.i of about 0.01. Infected cells may be harvested 30 to 60 hours post infection. Preferably, the cells are harvested 34 to 48 hours post infection. Still more preferably, the cells are harvested 38 to 40 hours post infection. Proteases (typically trypsin) are generally added during cell culture to allow viral release, and the proteases can be added at any suitable stage during the culture.

Haemagglutinin (HA) is the main immunogen in inactivated influenza vaccines, and vaccine doses are standardised by reference to HA levels, typically as measured by a single radial immunodiffution (SRID) assay. Vaccines typically contain about 15µg of HA per strain, although lower doses are also used *e.g*. for children, or in pandemic situations. Fractional doses such as ½ (*i.e*. 7.5 µg HA per strain), ¼ and ¹/₈ have been used [4,5], as have higher doses (*e.g*. 3x or 9x doses [138,139]).Thus vaccines may include between 0.1 and 150µg of HA per influenza strain, preferably between 0.1 and 50µg *e.g.* 0.1-20µg, 0.1-15µg, 0.1-10µg, 0.1-7.5µg, 0.5-5µg, *etc.* Particular doses include *e.g*. about 45, about 30, about 15, about 10, about 7.5, about 5, about 3.8, about 1.9, about 1.5, *etc.* These lower doses are most useful when an adjuvant is present in the vaccine, as with the invention.

For live vaccines, dosing is measured by median tissue culture infectious dose (TCID₅₀) rather than HA content, and a TCID₅₀ of between 10⁶ and 10⁸ (preferably between 10^{6.5}-10^{7.5}) per strain is typical.

HA used with the invention may be a natural HA as found in a virus, or may have been modified. For instance, it is known to modify HA to remove determinants (*e.g*. hyper-basic regions around the cleavage site between HA1 and HA2) that cause a virus to be highly pathogenic in avian species, as these determinants can otherwise prevent a virus from being grown in eggs.

Compositions of the invention may include detergent *e.g*. a polyoxyethylene sorbitan ester surfactant (known as 'Tweens'), an octoxynol (such as octoxynol-9 (Triton X-100) or t-octylphenoxypolyethoxyethanol), a cetyl trimethyl ammonium bromide ('CTAB'), or sodium deoxycholate, particularly for a split or surface antigen vaccine. The detergent may be present only at trace amounts. Thus the vaccine may included less than 1mg/ml of each of octoxynol-10, α-tocopheryl hydrogen succinate and polysorbate 80. Other residual components in trace amounts could be antibiotics (*e.g*. neomycin, kanamycin, polymyxin B).

An inactivated but non-whole cell vaccine (*e.g*. a split virus vaccine or a purified surface antigen vaccine) may include matrix protein, in order to benefit from the additional T cell epitopes that are located within this antigen. Thus a non-whole cell vaccine (particularly a split vaccine) that includes haemagglutinin and neuraminidase may additionally include M1 and/or M2 matrix protein. Where a matrix protein is present, inclusion of detectable levels of M2 matrix protein is preferred. Nucleoprotein may also be present.

### Pharmaceutical compositions

Compositions of the invention are pharmaceutically acceptable. They may include components in addition to the antigen, adjuvant and cytokine-inducing agent *e.g*. they will typically include one or more pharmaceutical carrier(s) and/or excipient(s). A thorough discussion of such components is available in reference 140.

The composition may include preservatives such as thiomersal or 2-phenoxyethanol. It is preferred, however, that the vaccine should be substantially free from (*i.e.* less than 5µg/ml) mercurial material e.g. thiomersal-free [88,141]. Vaccines containing no mercury are more preferred. Preservative-free vaccines are particularly preferred.

To control tonicity, it is preferred to include a physiological salt, such as a sodium salt. Sodium chloride (NaCl) is preferred, which may be present at between 1 and 20 mg/ml. Other salts that may be present include potassium chloride, potassium dihydrogen phosphate, disodium phosphate dehydrate, magnesium chloride, calcium chloride, *etc.*

Compositions will generally have an osmolality of between 200 mOsm/kg and 400 mOsm/kg, preferably between 240-360 mOsm/kg, and will more preferably fall within the range of 290-310 mOsm/kg. Osmolality has previously been reported not to have an impact on pain caused by vaccination [142], but keeping osmolality in this range is nevertheless preferred.

Compositions may include one or more buffers. Typical buffers include: a phosphate buffer; a Tris buffer; a borate buffer; a succinate buffer; a histidine buffer; or a citrate buffer. Buffers will typically be included in the 5-20mM range.

The pH of a composition will generally be between 5.0 and 8.1, and more typically between 6.0 and 8.0 *e.g*. between 6.5 and 7.5, or between 7.0 and 7.8. A process of the invention may therefore include a step of adjusting the pH of the bulk vaccine prior to packaging.

The composition is preferably sterile. The composition is preferably non-pyrogenic *e.g*. containing <1 EU (endotoxin unit, a standard measure) per dose, and preferably <0.1 EU per dose. The composition is preferably gluten free.

The composition may include material for a single immunisation, or may include material for multiple immunisations (*i.e*. a 'multidose' kit). The inclusion of a preservative is preferred in multidose arrangements. As an alternative (or in addition) to including a preservative in multidose compositions, the compositions may be contained in a container having an aseptic adaptor for removal of material.

Influenza vaccines are typically administered in a dosage volume of about 0.5ml, although a half dose (*i.e*. about 0.25ml) may be administered to children.

The antigen, emulsion and cytokine inducing agent in a composition will typically be in admixture.

Compositions and kits are preferably stored at between 2°C and 8°C. They should not be frozen. They should ideally be kept out of direct light.

### Kits of the invention

As mentioned above, compositions of the invention are preferably prepared extemporaneously, at the time of delivery. Thus the invention provides kits including the various components ready for mixing. The kit allows the oil-in-water emulsion and the antigen to be kept separately until the time of use. The cytokine-inducing agent may be included in one these two kit components, or may be part of a third kit component.

The components are physically separate from each other within the kit, and this separation can be achieved in various ways. For instance, the components may be in separate containers, such as vials. The contents of two vials can then be mixed *e.g*. by removing the contents of one vial and adding them to the other vial, or by separately removing the contents of both vials and mixing them in a third container.

In a preferred arrangement, one of the kit components is in a syringe and the other is in a container such as a vial. The syringe can be used (*e.g*. with a needle) to insert its contents into the second container for mixing, and the mixture can then be withdrawn into the syringe. The mixed contents of the syringe can then be administered to a patient, typically through a new sterile needle. Packing one component in a syringe eliminates the need for using a separate syringe for patient administration.

In another preferred arrangement, the two kit components are held together but separately in the same syringe *e.g*. a dual-chamber syringe, such as those disclosed in references 143-150 *etc.* When the syringe is actuated (*e.g*. during administration to a patient) then the contents of the two chambers are mixed. This arrangement avoids the need for a separate mixing step at the time of use.

The contents of the various kit components will generally all be in aqueous form. In some arrangements, a component (typically the antigen component rather than the emulsion component) is in dry form (*e.g*. in a lyophilised form), with the other component being in aqueous form. The two components can be mixed in order to reactivate the dry component and give an aqueous composition for administration to a patient. A lyophilised component will typically be located within a vial rather than a syringe. Dried components may include stabilizers such as lactose, sucrose or mannitol, as well as mixtures thereof *e.g*. lactose/sucrose mixtures, sucrose/mannitol mixtures, *etc.* One possible arrangement uses an aqueous emulsion component in a pre-filled syringe and a lyophilised antigen component in a vial.

### Packaging of compositions or kit components

Suitable containers for compositions of the invention (or kit components) include vials, syringes (*e.g.* disposable syringes), nasal sprays, *etc..* These containers should be sterile.

Where a composition/component is located in a vial, the vial is preferably made of a glass or plastic material. The vial is preferably sterilized before the composition is added to it. To avoid problems with latex-sensitive patients, vials are preferably sealed with a latex-free stopper, and the absence of latex in all packaging material is preferred. The vial may include a single dose of vaccine, or it may include more than one dose (a 'multidose' vial) *e.g*. 10 doses. Preferred vials are made of colorless glass.

A vial can have a cap (*e.g*. a Luer lock) adapted such that a pre-filled syringe can be inserted into the cap, the contents of the syringe can be expelled into the vial (*e.g*. to reconstitute lyophilised material therein), and the contents of the vial can be removed back into the syringe. After removal of the syringe from the vial, a needle can then be attached and the composition can be administered to a patient. The cap is preferably located inside a seal or cover, such that the seal or cover has to be removed before the cap can be accessed. A vial may have a cap that permits aseptic removal of its contents, particularly for multidose vials.

Where a component is packaged into a syringe, the syringe may have a needle attached to it. If a needle is not attached, a separate needle may be supplied with the syringe for assembly and use. Such a needle may be sheathed. Safety needles are preferred. 1-inch 23-gauge, 1-inch 25-gauge and 5/8-inch 25-gauge needles are typical. Syringes may be provided with peel-off labels on which the lot number, influenza season and expiration date of the contents may be printed, to facilitate record keeping. The plunger in the syringe preferably has a stopper to prevent the plunger from being accidentally removed during aspiration. The syringes may have a latex rubber cap and/or plunger. Disposable syringes contain a single dose of vaccine. The syringe will generally have a tip cap to seal the tip prior to attachment of a needle, and the tip cap is preferably made of a butyl rubber. If the syringe and needle are packaged separately then the needle is preferably fitted with a butyl rubber shield. Preferred syringes are those marketed under the trade name "Tip-Lok"™.

Containers may be marked to show a half-dose volume *e.g*. to facilitate delivery to children. For instance, a syringe containing a 0.5ml dose may have a mark showing a 0.25ml volume.

Where a glass container (*e.g*. a syringe or a vial) is used, then it is preferred to use a container made from a borosilicate glass rather than from a soda lime glass.

A kit or composition may be packaged (*e.g*. in the same box) with a leaflet including details of the vaccine *e.g.* instructions for administration, details of the antigens within the vaccine, *etc.* The instructions may also contain warnings *e.g*. to keep a solution of adrenaline readily available in case of anaphylactic reaction following vaccination, *etc.*

### Methods of treatment, and administration of the vaccine

Compositions of the invention are suitable for administration to human patients, and the invention provides a method of raising an immune response in a patient, comprising the step of administering a composition of the invention to the patient.

The invention also provides a kit or composition of the invention for use as a medicament.

The invention also provides the use of (i) an influenza virus antigen; (ii) an oil-in-water emulsion adjuvant; and (iii) a cytokine-inducing agent, in the manufacture of a medicament for raising an immune response in a patient.

The immune response raised by these methods and uses will generally include an antibody response, preferably a protective antibody response. Methods for assessing antibody responses, neutralising capability and protection after influenza virus vaccination are well known in the art. Human studies have shown that antibody titers against hemagglutinin of human influenza virus are correlated with protection (a serum sample hemagglutination-inhibition titer of about 30-40 gives around 50% protection from infection by a homologous virus) [151]. Antibody responses are typically measured by hemagglutination inhibition, by microneutralisation, by single radial immunodiffusion (SRID), and/or by single radial hemolysis (SRH). These assay techniques are well known in the art.

Compositions of the invention can be administered in various ways. The most preferred immunisation route is by intramuscular injection (*e.g*. into the arm or leg), but other available routes include subcutaneous injection, intranasal [152-154], oral [155], intradermal [156,157], transcutaneous, transdermal [158], *etc.*

Vaccines prepared according to the invention may be used to treat both children and adults. Influenza vaccines are currently recommended for use in pediatric and adult immunisation, from the age of 6 months. Thus the patient may be less than 1 year old, 1-5 years old, 5-15 years old, 15-55 years old, or at least 55 years old. Preferred patients for receiving the vaccines are the elderly (*e.g*. ≥50 years old, ≥60 years old, preferably ≥65 years), the young (*e.g.* ≤5 years old), hospitalised patients, healthcare workers, armed service and military personnel, pregnant women, the chronically ill, immunodeficient patients, patients who have taken an antiviral compound (*e.g*. an oseltamivir or zanamivir compound, such as oseltamivir phosphate; see below) in the 7 days prior to receiving the vaccine, and people travelling abroad. The vaccines are not suitable solely for these groups, however, and may be used more generally in a population. For pandemic strains, administration to all age groups is preferred.

Preferred compositions of the invention satisfy 1, 2 or 3 of the CPMP criteria for efficacy. In adults (18-60 years), these criteria are: (1) ≥70% seroprotection; (2) ≥40% seroconversion; and/or (3) a GMT increase of ≥2.5-fold. In elderly (>60 years), these criteria are: (1) ≥60% seroprotection; (2) ≥30% seroconversion; and/or (3) a GMT increase of ≥2-fold. These criteria are based on open label studies with at least 50 patients.

Treatment can be by a single dose schedule or a multiple dose schedule. Multiple doses may be used in a primary immunisation schedule and/or in a booster immunisation schedule. In a multiple dose schedule the various doses may be given by the same or different routes *e.g*. a parenteral prime and mucosal boost, a mucosal prime and parenteral boost, *etc.* Administration of more than one dose (typically two doses) is particularly useful in immunologically naïve patients *e.g*. for people who have never received an influenza vaccine before, or for vaccinating against a new HA subtype (as in a pandemic outbreak). Multiple doses will typically be administered at least 1 week apart (*e.g*. about 2 weeks, about 3 weeks, about 4 weeks, about 6 weeks, about 8 weeks, about 10 weeks, about 12 weeks, about 16 weeks, *etc*.).

Vaccines of the invention may be administered to patients at substantially the same time as *(e.g.* during the same medical consultation or visit to a healthcare professional) other vaccines *e.g*. at substantially the same time as a measles vaccine, a mumps vaccine, a rubella vaccine, a MMR vaccine, a varicella vaccine, a MMRV vaccine, a diphtheria vaccine, a tetanus vaccine, a pertussis vaccine, a DTP vaccine, a conjugated *H.influenzae* type b vaccine, an inactivated poliovirus vaccine, a hepatitis B virus vaccine, a meningococcal conjugate vaccine (such as a tetravalent A-C-W135-Y vaccine), a respiratory syncytial virus vaccine, a pneumococcal conjugate vaccine, *etc.* Administration at substantially the same time as a pneumococcal vaccine and/or a meningococcal vaccine is particularly useful in elderly patients.

Similarly, vaccines of the invention may be administered to patients at substantially the same time as (*e.g*. during the same medical consultation or visit to a healthcare professional) an antiviral compound, and in particular an antiviral compound active against influenza virus (*e.g*. oseltamivir and/or zanamivir). These antivirals include neuraminidase inhibitors, such as a (3R,4R,5S)-4-acetylamino-5-amino-3(1-ethylpropoxy)-1-cyclohexene-1-carboxylic acid or 5-(acetylamino)-4-[(aminoiminomethyl)-amino]-2,6-anhydro-3,4,5-trideoxy-D-glycero-D-galactonon-2-enonic acid, including esters thereof (*e.g*. the ethyl esters) and salts thereof (*e.g*. the phosphate salts). A preferred antiviral is (3R,4R,5S)-4-acetylamino-5-amino-3(1-ethylpropoxy)-1-cyclohexene-1-carboxylic acid, ethyl ester, phosphate (1:1), also known as oseltamivir phosphate (TAMIFLU™).

### General

The term "comprising" encompasses "including" as well as "consisting" *e.g*. a composition "comprising" X may consist exclusively of X or may include something additional *e.g*. X + Y.

The word "substantially" does not exclude "completely" *e.g*. a composition which is "substantially free" from Y may be completely free from Y. Where necessary, the word "substantially" may be omitted from the definition of the invention.

The term "about" in relation to a numerical value *x* means, for example, *x*±10%.

Unless specifically stated, a process comprising a step of mixing two or more components does not require any specific order of mixing. Thus components can be mixed in any order. Where there are three components then two components can be combined with each other, and then the combination may be combined with the third component, *etc*.

Where animal (and particularly bovine) materials are used in the culture of cells, they should be obtained from sources that are free from transmissible spongiform encaphalopathies (TSEs), and in particular free from bovine spongiform encephalopathy (BSE). Overall, it is preferred to culture cells in the total absence of animal-derived materials.

Where a compound is administered to the body as part of a composition then that compound may alternatively be replaced by a suitable prodrug.

Where a cell substrate is used for reassortment or reverse genetics procedures, it is preferably one that has been approved for use in human vaccine production *e.g*. as in Ph Eur general chapter 5.2.3.

### BRIEF DESCRIPTION OF DRAWINGS

Figures 1 to 3 show the Log10 serum antibody titers (ELISA) for mice immunized with different compositions. Arrows show compositions that included the MF59 emulsion. From left to right, the bars are grouped as follows: the four adjuvants (i) to (iv) alone; the four CpG combinations; the four R-848 combinations; the four ER-57 combinations; a control with no additives; and the two components (a) and (b) alone. Thus the left-most arrow shows results for MF59 alone.
Figure 4 shows the percentage of CD4⁺ T cells that gave an antigen-specific cytokine response when stimulated by HA (left bar in each pair) and the percentage that were γ-interferon positive (right bar in each pair). The groups on the X-axis are as in Figures 1 to 3.
Figure 5 shows GMTs (AU/ml) for IgG against the H3N2 strain. The left bar in each pair shows IgG1; the right shows IgG2a.
Figure 6 shows serum anti-HA ELISA responses (after 2 doses) in mice receiving trivalent egg-grown antigens. Experiments were with no adjuvant, or with MF59 and/or CpG7909. Figure 7 similarly shows anti-HA HI responses in the same mice. Figure 8 shows the proportion anti-H3N2 IgG1 and IgG2a, assessed by ELISA.
Figure 9 shows the number of cytokine positive cells, as a % of total CD4+ cells. Responses from two individual mice are shown. Mice were immunized with split vaccines "A" or "B", either unadjuvanted or adjuvanted with adjuvants (1), (2) or (3).

### MODES FOR CARRYING OUT THE INVENTION

Influenza virus strains Wyoming H3N2 (A), New-Caledonia H1N1 (A) and Jiangsu (B) were separately grown on MDCK cells. A trivalent surface glycoprotein vaccine was prepared and was used to immunize immune-naïve Balb/C mice at two doses (0.1 and 1 µg HA per strain) at days 0 and 28. Animals were bled at day 42 and various assays were performed with the blood: HI titers; anti-HA responses, measured by ELISA; and the level of CD4⁺ T cells that release cytokines in an antigen-specific manner, including a separate measurement of those that release γ-interferon. IgG responses were measured specifically in respect of IgG1 and IgG2a.

Compositions used for immunization (except for negative controls) included one of: (i) MF59 emulsion, mixed at a 1:1 volume ratio with the antigen solution; (ii) an aluminum hydroxide, used at 1mg/ml and including a 5mM histidine buffer; (iii) calcium phosphate, used at 1mg/ml and including a 5mM histidine buffer; or (iv) microparticles formed from poly(lactide co-glycolide) 50:50 copolymer composition, intrinsic viscosity 0.4 ('PLG'), with adsorbed antigen. In addition (again, except for negative controls) the compositions included one of: (a) an immunostimulatory CpG ODN with a phosphorothioate backbone; or (b) R-848.

Testing each of these six components separately, only the MF59 emulsion gave consistently useful increases in HI titers for all three strains at both doses. For the H1N1 strain, titers were >10-fold higher than in the unadjuvanted control. The increase for the H3N2 strain was >5-fold at the lower antigen dose but >10-fold at the higher dose. The increase for the influenza B virus strain was >3-fold at the lower antigen dose but >5-fold at the higher dose.

Looking at the combinations then, for the influenza B virus, only two combinations increased the HI titer at day 42 by more than 3-fold (relative to the unadjuvanted control vaccine) when using 0.1µg antigen, and these were the two MF59-based combinations.

For the H1N1 strain then all of the combinations with CpG, except for the CpG/PLG combination, gave at least a 5-fold increase in HI titers, and the increase when using the MF59/CpG combination was more than 10-fold. The other MF59-based combination showed a >5-fold increase.

For the H3N2 strain then, again, all of the combinations with CpG (except for the CpG/PLG combination, which gave a >3-fold increase) gave at least a 5-fold increase in HI titers. The MF59/R-848 and Alum/R-848 combinations gave a >3-fold increase.

Overall, therefore, the best adjuvant for increasing HI titers from options (i) to (iv) was the oil-in-water emulsion. The better additive from (a) or (b) was CpG, although CpG alone did not enhance HI titers. The best combinations were all based on the oil-in-water emulsion.

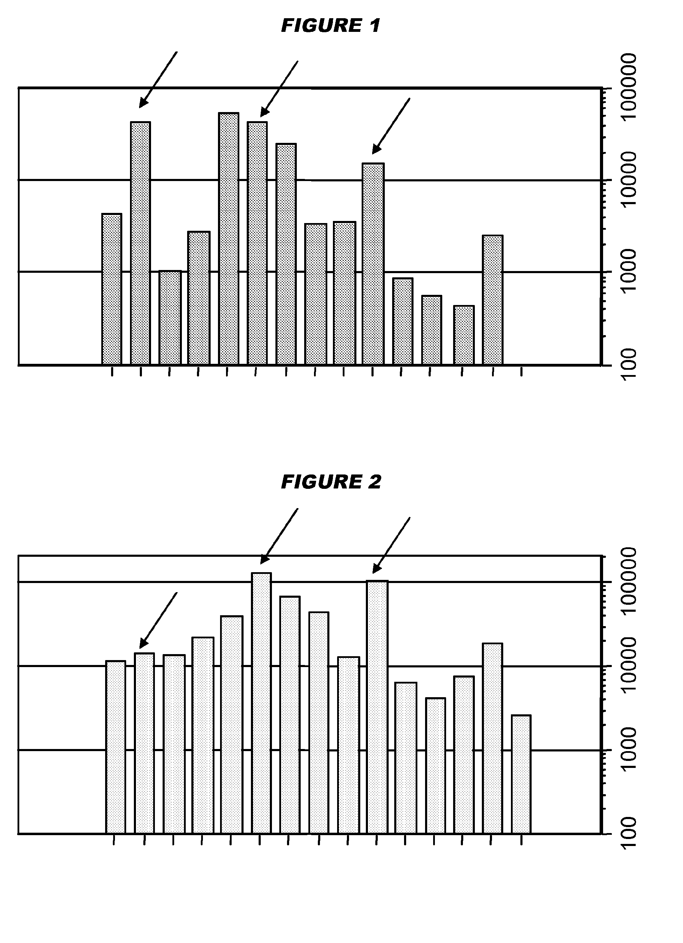

Figures 1 to 3 show anti-HA ELISA responses for the 15 groups: 1 with no adjuvant; 3 with (a) and (b); 4 with (i) to (iv); and 8 with the combinations of (i)-(iv)/(a)-(b). The arrows show the three compositions that include the MF59 oil-in-water emulsion. It is immediately apparent that the emulsion-based compositions gave the best anti-HA responses.

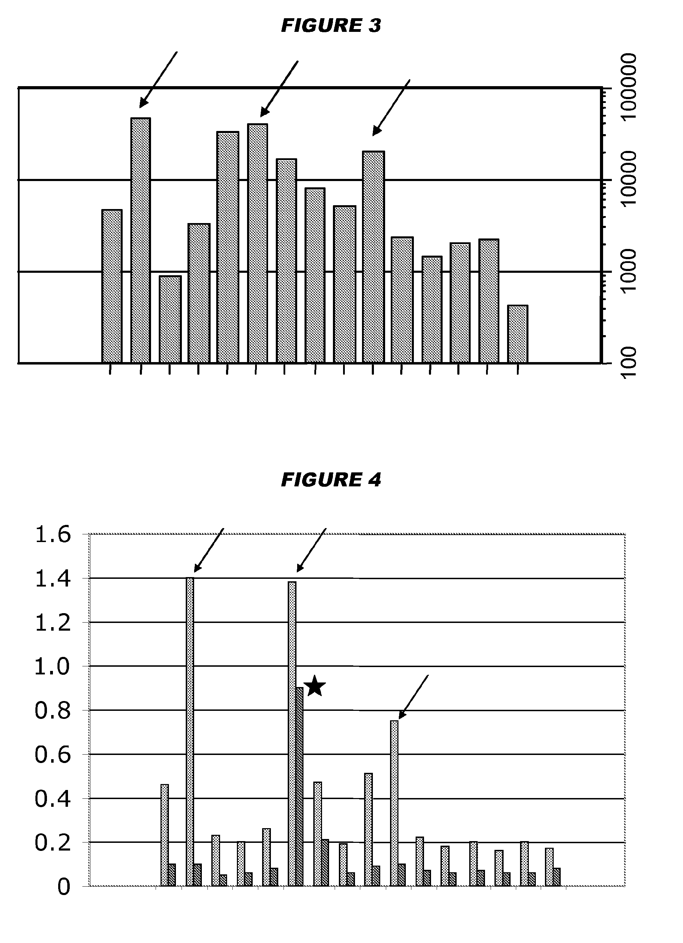

Figure 4 shows the adjuvants that gave the best T cell responses. Again, it is immediately apparent that the emulsion-based compositions gave the best responses. For (a) and (b) alone, T cell responses were modest, and the best results were seen when they were combined with MF59. The highest level of γ-interferon-secreting cells was achieved with the MF59/CpG combination, marked with a star.

The number of γ-interferon-secreting cells was better with the MF59/CpG combination than with either of the components on its own.

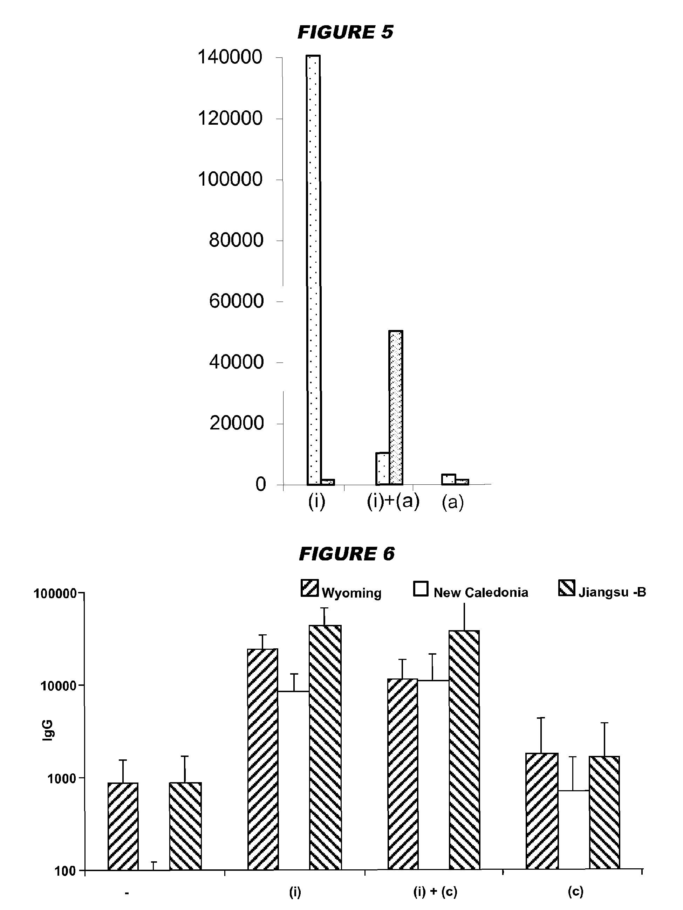

The increase in γ-interferon secretion shows that, whereas the MF59 adjuvant alone elicited a mainly Th2-type response, the addition of CpG shifted the response towards a Th1-type. Th1-type responses have been reported to improve heterosubtypic immunity [159]. The shift towards a Th1-type response was also seen when IgG types were examined. As shown in Figure 5, MF59 alone shows a strong IgG1 response (Th2) and a low IgG2a response (Th1). CpG shows weak IgG1 and IgG2a responses. In contrast, the MF59/CpG combination shows a dominant IgG2a response.

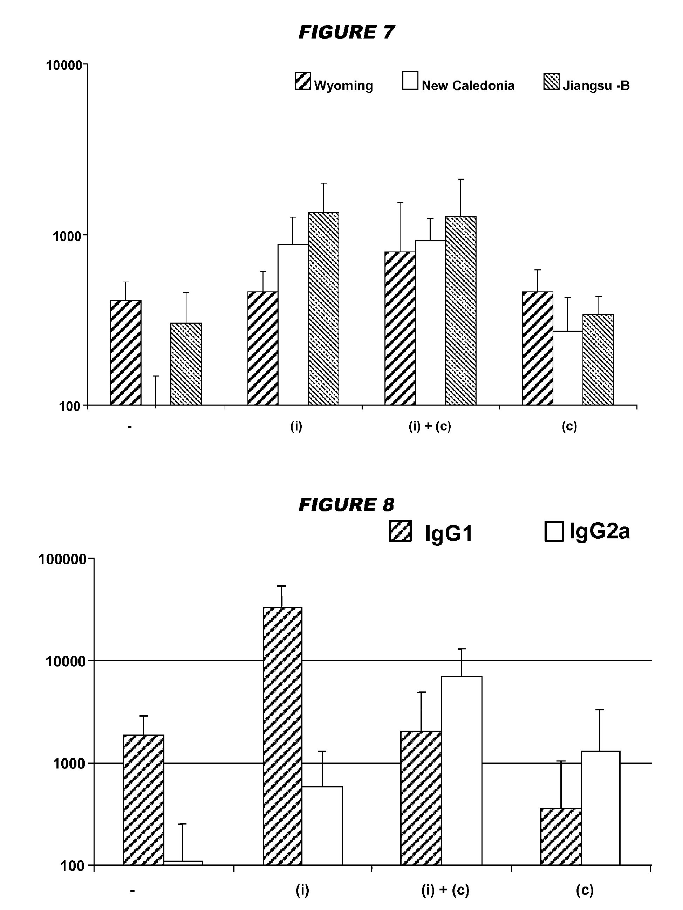

In further experiments, using purified surface glycoproteins prepared from viruses grown on eggs, the MF59/CpG combination was modified to use a different immunostimulatory oligonucleotide, namely (c) CpG7909. As shown in Figures 6 to 8, the results obtained in these experiments were identical to the previous ones. In particular, Figure 6 shows that anti-HA serum ELISA IgG titers were dramatically increased by the addition of MF59 to the antigens, whereas the addition of CpG7909 alone did not lead to a comparable enhancement. Similarly, the titers obtained with MF59 were not significantly further increased by the addition of CpG7909. Essentially the same pattern is seen with serum HI titers (Figure 7). When looking at the quality of the antibody response, however, the addition of CpG7909 increases the relative contribution of the Th1-associated isotype (Figure 8).

Antibody data correlated well with the cytokine profiles of CD4 T cells responding specifically to antigen restimulation. Again, MF59 led to an increase in the frequency of Ag-responding T cells. Addition of CpG7909 did not greatly increase the overall percentage of responding T cells but changes the composition of cytokines produced by these responding cells. Thus, a higher proportion of Ag-specific T cells produced IFN-γ when CpG7909 is included, whereas a lower proportion of them produced IL-5.

In additional experiments, two commercially available unadjuvanted split virion trivalent influenza vaccines ("SPLIT (A)" and "SPLIT (B)") were obtained and used to immunize mice. The vaccines were diluted to give a dose of 0.2µg each HA. Vaccines were either unadjuvanted, or were adjuvanted with (1) aluminium hydroxide, (2) MF59 emulsion, or (3) MF59 emulsion and an immunostimulatory CpG oligonucleotide. Groups of 8 female Balb/C mice, 8 weeks old, were immunized intramuscularly with the vaccines, with 50µl doses on days 0 and 28. Sera were obtained on days 14 and 42, and were analysed for anti-HA titer (IgG), HI titer and T cells.

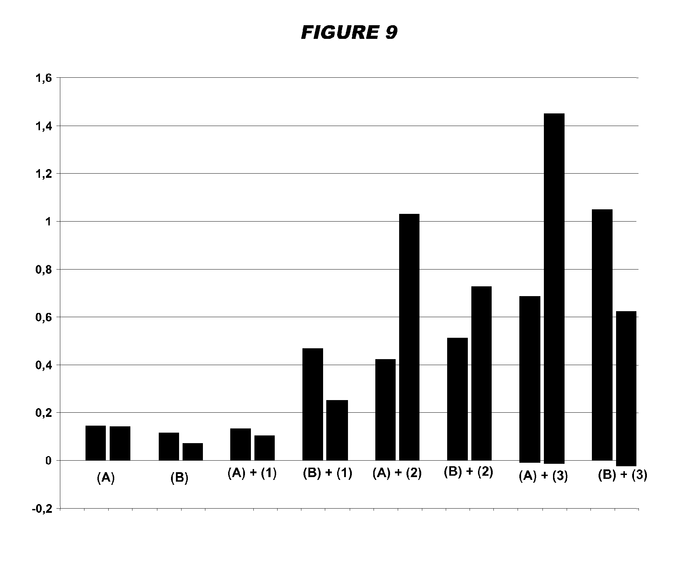

Serum IgG antibody titers (ELISA) at day 42 are given in Table I below, looking at each virus separately. HI serum antibody titers are in Table II. Figure 9 shows T cell responses in the mice. As seen with the purified surface glycoprotein vaccines, MF59 gave better results than alum, but the addition of the CpG oligonucleotide to MF59 led in general to better T cell responses. For instance, adding CpG to MF59 "split (A)" resulted in a higher proportion of antigen-specific T cells than achieved with MF59 alone.

Thus oil-in-water emulsions are excellent adjuvants for influenza vaccines, including both surface glycoprotein vaccines and split vaccines, but their ability to elicit cytokine responses, in particular γ-interferon responses, can be improved by additionally including an immunostimulating agent such as CpG.

Oil-in-water emulsions offer enhanced neutralization of heterovariant influenza strains, such that a vaccine may induce protective immunity even if the vaccine strain does not match the circulating strain [160]. It has now been shown that addition of a cytokine-inducing agent can give a vaccine where good HI titers are maintained, and in which T cell and cytokine responses are enhanced. HI titers correlate with serum neutralization of influenza virus, and so maintaining high HI titers is useful, particularly for strains to which a population is naive or which can evade host cytokine responses [161]. The increased T cell and cytokine responses are useful because they are involved in the early and decisive stages of host defense against influenza infection [7], and it may be possible to diminish age-related susceptibility to influenza by inducing a more potent interferon-γ response [23]. Thus the combination of an oil-in-water emulsion and a cytokine-inducing agent is advantageous.

The invention further provides the following non-limiting embodiments:
1. An immunogenic composition comprising: (i) an influenza virus antigen; (ii) an oil-in-water emulsion adjuvant; and (iii) a cytokine-inducing agent.
2. The composition of embodiment 1, wherein the influenza virus antigen is inactivated virus.
3. The composition of embodiment 1, wherein the influenza virus antigen comprises whole virus, split virus, or purified surface antigens.
4. The composition of any preceding embodiment, wherein the influenza virus antigen is from a H1, H2, H3, H5, H7 or H9 influenza A virus subtype.
5. The composition of any preceding embodiment, wherein the influenza virus antigen is prepared from an influenza virus grown on eggs.
6. The composition of any preceding embodiment, wherein the influenza virus antigen is prepared from an influenza virus grown on cell culture.
7. The composition of any one of embodiments 1 to 4, wherein the composition is free from ovalbumin, ovomucoid and chicken DNA.
8. The composition of embodiment 6 or embodiment 7, wherein the composition contains less than 10ng of cellular DNA from the cell culture host.
9. The composition of embodiment 6 or embodiment 7, wherein the composition contains less than 10ng of DNA that is 100 nucleotides or longer.
10. The composition of any preceding embodiment, wherein the influenza virus antigen is prepared from an influenza virus having one or more RNA segments from an A/PR/8/34 influenza virus.
11. The composition of any preceding embodiment, wherein the influenza virus antigen is prepared from an influenza virus obtained by reverse genetics techniques.
12. The composition of any one of embodiments 6 to 11, wherein the cell culture is a microcarrier culture, an adherent culture, or a suspension culture.
13. The composition of any one of embodiments 6 to 12, wherein the cell culture is serum-free.
14. The composition of any one of embodiments 6 to 13, wherein the influenza virus antigen is prepared from an influenza virus grown on MDCK cells.
15. The composition of any preceding embodiment, wherein the composition contains between 0.1 and 20µg of haemagglutinin per viral strain.
16. The composition of any preceding embodiment, wherein the oil(s) and surfactant(s) in the emulsion are biodegradable and biocompatible.
17. The composition of any preceding embodiment, wherein the emulsion has droplets with a sub-micron diameter.
18. The composition of any preceding embodiment, wherein the emulsion includes a terpenoid.
19. The composition of any preceding embodiment, wherein the emulsion includes squalene.
20. The composition of any preceding embodiment, wherein the emulsion includes a tocopherol.
21. The composition of embodiment 20, wherein the tocopherol is DL-α-tocopherol.
22. The composition of any preceding embodiment, wherein the emulsion includes a polyoxyethylene sorbitan esters surfactant, a octoxynol surfactant, and/or a sorbitan ester.
23. The composition of any preceding embodiment, wherein the cytokine-inducing agent elicits the release of interferon-γ.
24. The composition of any preceding embodiment, wherein the cytokine-inducing agent is an agonist of one or more of the human TLR1, TLR2, TLR3, TLR4, TLR7, TLR8, and/or TLR9.
25. The composition of any preceding embodiment, wherein the cytokine-inducing agent is selected from: an immunostimulatory oligonucleotide; a 3-O-deacylated monophosphoryl lipid A (3dMPL); an imidazoquinoline compound; and/or an aminoalkyl glucosaminide phosphate derivative.
26. The composition of embodiment 25, wherein the cytokine-inducing agent is 3dMPL, and where at least 10% by weight of the 3dMPL is the hexaacyl chain form.
27. The composition of embodiment 25 or embodiment 26, wherein the cytokine-inducing agent is 3dMPL, and where the 3dMPL is in the form of particles with a diameter <150nm.
28. The composition of embodiment 25 or embodiment 26 or embodiment 27, wherein the cytokine-inducing agent is 3dMPL, and where the 3dMPL is located in the aqueous phase of the emulsion.
29. The composition of any preceding embodiment, being substantially free from mercurial material.
30. The composition of any preceding embodiment, including between 1 and 20 mg/ml sodium chloride.
31. The composition of any preceding embodiment, having an osmolality between 200 and 400 mOsm/kg.
32. The composition of any preceding embodiment, including one or more buffer(s).
33. The composition of embodiment 32, wherein the buffer(s) include: a phosphate buffer; a Tris buffer; a borate buffer; a succinate buffer; a histidine buffer; or a citrate buffer.
34. The composition of any preceding embodiment, having a pH between 5.0 and 8.1.
35. The composition of any preceding embodiment, containing <1 endotoxin unit per dose.
36. The composition of any preceding embodiment, being gluten free.
37. The composition of any preceding embodiment, wherein the composition includes two influenza A strains and one influenza B strain.
38. The composition of any one of embodiments 1 to 36, wherein the composition is a monovalent vaccine against a pandemic influenza virus strain.
39. A method for preparing an immunogenic composition comprising the steps of combining: (i) an influenza virus antigen; (ii) an oil-in-water emulsion adjuvant; and (iii) a cytokine inducing agent.
40. A kit comprising: (i) a first kit component comprising an influenza virus antigen; and (ii) a second kit component comprising an oil-in-water emulsion adjuvant, wherein either (a) the first component or the second component includes a cytokine inducing agent, or (b) the kit includes a third kit component comprising a cytokine inducing agent.
41. The kit of embodiment 40, wherein the first component and the second component are in separate containers.
42. The kit of embodiment 41, wherein the first and second components are in vials.
43. The kit of embodiment 41, wherein one of the first and second components is in a syringe, and wherein the other component is in a vial.
44. The kit of embodiment 42 or embodiment 43, wherein the vial is made of a glass or plastic material.
45. The kit of embodiment 42, embodiment 43 or embodiment 44, wherein the vial is sealed with a latex-free stopper.
46. A method of raising an immune response in a patient, comprising the step of administering to the patient a medicament, wherein the medicament is a composition of any one of embodiments 1 to 35.
47. The use of (i) an influenza virus antigen; (ii) an oil-in-water emulsion adjuvant; and (iii) a cytokine inducing agent, in the manufacture of a medicament for raising an immune response in a patient.
48. The method of embodiment 46, or the use of embodiment 47, wherein the medicament is administered to a patient at substantially the same time as a pneumococcal conjugate vaccine.
49. The method of embodiment 46, or the use of embodiment 47, wherein the medicament is administered to a patient at substantially the same time as an antiviral compound active against influenza virus.

It will be understood that the invention has been described by way of example only and modifications may be made whilst remaining within the scope and spirit of the invention.

**Table I**

| | **Unadjuvanted** | **Alum** | **MF59** | **MF59+CpG** |
|---|---|---|---|---|
| ***Anti-H1N1*** | | | | |
| SPLIT (A) | 749 | 1329 | 7690 | 8808 |
| SPLIT (B) | 1175 | 1991 | 7738 | 6754 |

| ***Anti-H3N2*** | | | | |
|---|---|---|---|---|
| SPLIT (A) | 412 | 977 | 4583 | 6032 |
| SPLIT (B) | 1111 | 1465 | 6005 | 5308 |

| ***Anti-B*** | | | | |
|---|---|---|---|---|
| SPLIT (A) | 707 | 2534 | 8716 | 11211 |
| SPLIT (B) | 1585 | 2520 | 13682 | 10837 |

**Table II**

| | **Unadjuvanted** | **Alum** | **MF59** | **MF59+CpG** |
|---|---|---|---|---|
| ***Anti-H1N1*** | | | | |
| SPLIT (A) | 140 | 280 | 800 | 1387 |
| SPLIT (B) | 285 | 330 | 1300 | 1371 |

| ***Anti-H3N2*** | | | | |
|---|---|---|---|---|
| SPLIT (A) | 290 | 370 | 510 | 1863 |
| SPLIT (B) | 380 | 390 | 460 | 960 |

| ***Anti-B*** | | | | |
|---|---|---|---|---|
| SPLIT (A) | 280 | 780 | 1560 | 800 |
| SPLIT (B) | 550 | 440 | 2280 | 1371 |

### REFERENCES (the contents of which are hereby incorporated by reference)

[1] Vaccines. (eds. Plotkin & Orenstein). 4th edition, 2004, ISBN: 0-7216-9688-0.
[2] US 6,372,223.
[3] WO00/15251.
[4] WO01/22992.
[5] Hehme et al. (2004) Virus Res. 103(1-2):163-71.
[6] Frey et al. (2003) Vaccine 21:4234-7.
[7] Hayden et al. (1998) J Clin Invest 101(3):643-9.
[8] WO90/14837.
[9] Podda & Del Giudice (2003) Expert Rev vaccines 2:197-203.
[10] Podda (2001) Vaccine 19: 2673-2680.
[11] Vaccine Design: The Subunit and Adjuvant Approach (eds. Powell & Newman) Plenum Press 1995 (ISBN 0-306-44867-X).
*[12]* Vaccine Adjuvants: Preparation Methods and Research Protocols (Volume 42 of Methods in Molecular Medicine series). ISBN: 1-59259-083-7. Ed. O'Hagan.
[13] Allison & Byars (1992) Res Immunol 143:519-25.
[14] Hariharan et al. (1995) Cancer Res 55:3486-9.
[15] WO95/11700.
[16] US patent 6,080,725.
[17] W02005/097181.
[18] W02006/113373.
[19] Han et al. (2005) Impact of Vitamin E on Immune Function and Infectious Diseases in the Aged at Nutrition, Immune functions and Health EuroConference, Paris, 9-10 June 2005.
[20] Han et al. (2004) Ann N YAcad Sci 1031:96-101.
[21] US- 6630161.
[22] Powers & Belshe (1993) J Infect Dis 167:584-92.
[23] Mbawuike et al. (1996) Cell Immunol 173:64-78.
[24] Tassignon et al. (2005) J Immunol Meth 305:188-98.
[25] Myers et al. (1990) pages 145-156 of Cellular and molecular aspects of endotoxin reactions*.*
[26] Ulrich (2000) Chapter 16 (pages 273-282) of reference 12.
[27] Johnson et al. (1999) J Med Chem 42:4640-9.
[28] Baldrick et al. (2002) Regulatory Toxicol Pharmacol 35:398-413.
[29] US 4,680,338.
[30] US 4,988,815.
[31] WO92/15582.
[32] Stanley (2002) Clin Exp Dermatol 27:571-577.
[33] Wu et al. (2004) Antiviral Res. 64(2):79-83.
[34] Vasilakos et al. (2000) Cell Immunol. 204(1):64-74.
[35] US patents 4689338, 4929624, 5238944, 5266575, 5268376, 5346905, 5352784, 5389640, 5395937, 5482936, 5494916, 5525612, 6083505, 6440992, 6627640, 6656938, 6660735, 6660747, 6664260, 6664264, 6664265, 6667312, 6670372, 6677347, 6677348, 6677349, 6683088, 6703402, 6743920, 6800624, 6809203, 6888000 and 6924293.
[36] Jones (2003) Curr Opin Investig Drugs 4:214-218.
[37] W02004/060308.
[38] W02004/064759.
[39] US 6,924,271.
[40] US2005/0070556.
[41] US 5,658,731.
[42] US 5,011,828.
[43] W02004/87153.
[44] US 6,605,617.
[45] WO02/18383.
[46] WO2004/018455.
[47] WO03/082272.
[48] Dyakonova et al. (2004) Int Immunopharmacol 4(13):1615-23.
[49] FR-2859633.
[50] WO2006/002422.
[51] WO03/011223.
[52] Johnson et al. (1999) Bioorg Med Chem Lett 9:2273-2278.
[53] Evans et al. (2003) Expert Rev Vaccines 2:219-229.
[54] Thompson et al. (2005) J Leukoc Biol 78: 'The low-toxicity versions of LPS, MPLC adjuvant and RC529, are efficient adjuvants for CD4+ T cells'.
[55] Andrianov et al. (1998) Biomaterials 19:109-115.
[56] Payne et al. (1998) Adv Drug Delivery Review 31:185-196.
[57] Wong et al. (2003) J Clin Pharmacol 43(7):735-42.
[58] US2005/0215517.
[59] Thompson et al. (2003) Methods in Molecular Medicine 94:255-266.
[60] Kandimalla et al. (2003) Nucleic Acids Research 31:2393-2400.
[61] WO02/26757.
[62] WO99/62923.
[63] Krieg (2003) Nature Medicine 9:831-835.
[64] McCluskie et al. (2002) FEMS Immunology and Medical Microbiology 32:179-185.
[65] WO98/40100.
[66] US 6,207,646.
[67] US 6,239,116.
[68] US 6,429,199.
[69] Kandimalla et al. (2003) Biochemical Society Transactions 31 (part 3):654-658.
[70] Blackwell et al. (2003) J Immunol 170:4061-4068.
[71] Krieg (2002) Trends Immunol 23:64-65.
[72] WO01/95935.
[73] Kandimalla et al. (2003) BBRC 306:948-953.
[74] Bhagat et al. (2003) BBRC 300:853-861.
[75] WO03/035836.
[76] WO01/22972.
[77] UK patent application GB-A-2220211.
[78] WO 94/21292.
[79] WO94/00153.
[80] WO95/17210.
[81] WO96/26741.
[82] WO93/19780.
[83] WO96/37624.
[84] WO98/46262.
[85] WO02/28422.
[86] WO02/067983.
[87] WO02/074336.
[88] WO02/097072.
[89] WO2005/113756.
[90] WO01/21151.
[91] Huckriede et al. (2003) Methods Enzymol 373:74-91.
[92] Herlocher et al. (2004) J Infect Dis 190(9):1627-30.
[93] Le et al. (2005) Nature 437(7062):1108.
[94] World Health Organisation (2005) Emerging lnfectious Diseases 11(10):1515-21.
[95] Hoffmann et al. (2002) Vaccine 20:3165-3170.
[96] Subbarao et al. (2003) Virology 305:192-200.
[97] Liu et al. (2003) Virology 314:580-590.
[98] Ozaki et al. (2004) J. Virol. 78:1851-1857.
[99] Webby et al. (2004) Lancet 363:1099-1103.
[100] WO00/60050.
[101] WO01/04333.
[102] US 6649372.
[103] Neumann et al. (2005) Proc Natl Acad Sci USA 102:16825-9.
[104] W02006/067211.
[105] WO01/83794.
[106] Hoffmann et al. (2000) Virology 267(2):310-7.
[107] WO97/37000.
[108] Brands et al. (1999) Dev Biol Stand 98:93-100.
[109] Halperin et al. (2002) Vaccine 20:1240-7.
[110] Tree et al. (2001) Vaccine 19:3444-50.
[111] Kistner et al. (1998) Vaccine 16:960-8.
[112] Kistner et al. (1999) Dev Biol Stand 98:101-110.
[113] Bruhl et al. (2000) Vaccine 19:1149-58.
[114] Pau et al. (2001) Vaccines 19:2716-21.
[115] *http:*//*www. atcc. org*/
[116] *http:*//*locus.umdnj.edu*/
[117] WO3/076601.
[118] W02005/042728.
[119] WO03/043415.
[120] WO01/85938.
[121] WO2006/108846.
[122] EP-A-1260581 (WO01/64846).
[123] WO2006/071563.
[124] WO2005/113758.
[125] WO2006/027698.
[126] Lundblad (2001) Biotechnology and Applied Biochemistry 34:195-197.
*[127]* Guidance for Industry: Bioanalytical Method Validation. U.S. Department of Health and Human Services Food and Drug Administration Center for Drug Evaluation and Research (CDER) Center for Veterinary Medicine (CVM). May 2001.
[128] Ji et al. (2002) Biotechniques. 32:1162-7.
[129] Briggs (1991) J Parenter Sci Technol. 45:7-12.
[130] Lahijani et al. (1998) Hum Gene Ther. 9:1173-80.
[131] Lokteff et al. (2001) Biologicals. 29:123-32.
[132] EP-B-0870508.
[133] US 5948410.
[134] International patent application entitled "CELL-DERIVED VIRAL VACCINES WITH LOW LEVELS OF RESIDUAL CELL DNA", filed 1st November 2006 claiming priority US-60/732786.
[135] WO03/023021
[136] WO03/023025
[137] WO97/37001.
[138] Treanor et al. (1996) J Infect Dis 173:1467-70.
[139] Keitel et al. (1996) Clin Diagn Lab Immunol 3:507-10.
[140] Gennaro (2000) Remington: The Science and Practice of Pharmacy. 20th edition, ISBN: 0683306472.
[141] Banzhoff (2000) Immunology Letters 71:91-96.
[142] Nony et al. (2001) Vaccines 27:3645-51.
[143] W02005/089837.
[144] US 6,692,468.
[145] WO00/07647.
[146] WO99/17820.
[147] US 5,971,953.
[148] US 4,060,082.
[149] EP-A-0520618.
[150] WO98/01174.
[151] Potter & Oxford (1979) Br Med Bull 35: 69-75.
[152] Greenbaum et al. (2004) Vaccine 22:2566-77.
[153] Zurbriggen et al. (2003) Expert Rev Vaccines 2:295-304.
[154] Piascik (2003) J Am Pharm Assoc (Wash DC). 43:728-30.
[155] Mann et al. (2004) Vaccine 22:2425-9.
[156] Halperin et al. (1979) Am J Public Health 69:1247-50.
[157] Herbert et al. (1979) J Infect Dis 140:234-8.
[158] Chen et al. (2003) Vaccine 21:2830-6.
[159] Moran et al. (1999) J Infect Dis 180:579-85.
[160] Stephenson et al. (2005) J Infect Dis 191(8):1210-5.
[161] Seo et al. (2002) Nat Med 8(9):950-4.

## Claims

1. An immunogenic composition comprising: (i) an influenza virus antigen; (ii) an oil-in-water emulsion adjuvant; and (iii) a cytokine-inducing agent, wherein the cytokine inducing agent is an agonist of human TLR4.

2. The composition of claim 1, wherein the influenza virus antigen
(a) is inactivated virus; or
(b) comprises whole virus, split virus, or purified surface antigens

3. The composition of any preceding claim, wherein the influenza virus antigen is prepared from
(a) an influenza virus grown on eggs, or
(b) an influenza virus grown on cell culture.

4. The composition of any one of claims 1, 2 or 3(b), wherein the composition
(a) is free from ovalbumin, ovomucoid and chicken DNA; and/or
(b) contains less than 10ng of cellular DNA from the cell culture host; and/or
(c) contains less than 10ng of DNA that is 100 nucleotides or longer.

5. The composition of any preceding claim, wherein the influenza virus antigen is prepared from
(a) an influenza virus having one or more RNA segments from an A/PR/8/34 influenza virus; and/or
(b) an influenza virus obtained by reverse genetics techniques.

6. The composition of any one of claims 4 or 5, wherein the cell culture
(a) is a microcarrier culture, an adherent culture, or a suspension culture; and/or
(b) is serum-free; and/or
wherein the cell culture can be a MDCK cell culture.

7. The composition of any preceding claim, wherein the composition contains between 0.1 and 20µg of haemagglutinin per viral strain.

8. The composition of any preceding claim, wherein the oil(s) and surfactant(s) in the emulsion are biodegradable and biocompatible.

9. The composition of any preceding claim, wherein the emulsion has droplets with a sub-micron diameter.

10. The composition of any preceding claim, wherein the emulsion includes
(a) a terpenoid; and/or
(b) squalene; and/or
(c) a tocopherol, such as DL-α-tocopherol; and/or
(d) a polyoxyethylene sorbitan esters surfactant, a octoxynol surfactant, and/or a sorbitan ester.

11. The composition of any preceding claim, wherein the cytokine-inducing agent
(a) elicits the release of interferon-γ; and/or
(b) is selected from: an immunostimulatory oligonucleotide; a 3-O-deacylated monophosphoryl lipid A (3dMPL); an imidazoquinoline compound; and/or an aminoalkyl glucosaminide phosphate derivative.

12. The composition of claim 11(b), wherein the cytokine-inducing agent is 3dMPL, and where
(a) at least 10% by weight of the 3dMPL is the hexaacyl chain form; and/or
(b) the 3dMPL is in the form of particles with a diameter <150nm; amd/or
(c) the 3dMPL is located in the aqueous phase of the emulsion.

13. The composition of any preceding claim, wherein the composition
(a) is substantially free from mercurial material; and/or
(b) includes between 1 and 20 mg/ml sodium chloride; and/or
(c) has an osmolality between 200 and 400 mOsm/kg; and/or
(d) has a pH between 5.0 and 8.1; and/or
(e) contains <1 endotoxin unit per dose; and/or
(f) is gluten free; and/or
(g) includes one or more buffer(s), such as buffer(s) including a phosphate buffer; a Tris buffer; a borate buffer; a succinate buffer; a histidine buffer; or a citrate buffer.

14. The composition of any preceding claim, wherein the composition includes two influenza A strains and one influenza B strain or wherein the composition is a monovalent vaccine against a pandemic influenza virus strain.

15. A method for preparing an immunogenic composition comprising the steps of combining: (i) an influenza virus antigen; (ii) an oil-in-water emulsion adjuvant; and (iii) a cytokine inducing agent, wherein the cytokine inducing agent is an agonist of human TLR4.

16. A kit comprising: (i) a first kit component comprising an influenza virus antigen; and (ii) a second kit component comprising an oil-in-water emulsion adjuvant, wherein either (a) the first component or the second component includes a cytokine inducing agent, or (b) the kit includes a third kit component comprising a cytokine inducing agent, wherein the cytokine inducing agent is an agonist of human TLR4.

17. The kit of claim 16, wherein the first component and the second component are in separate containers; for example wherein the first and second components are in vials or wherein one of the first and second components is in a syringe, and wherein the other component is in a vial, wherein the vial is optionally made of a glass or plastic material and/or wherein the vial is sealed with a latex-free stopper.

18. A combination of (i) an influenza virus antigen; (ii) an oil-in-water emulsion adjuvant; and (iii) a cytokine inducing agent which is an agonist of human TLR4, for use in raising an immune response in a patient.
